(19) Europäisches Patentamt
European Patent Office
Office européen des brevets

(11) Publication number: **0 261 503 B1**

(12) # EUROPEAN PATENT SPECIFICATION

(45) Date of publication of patent specification: **15.04.92**

(51) Int. Cl.5: **C07D 277/46**, C07D 277/56, C07D 333/36, C07D 333/38, C07D 333/40, C07D 409/04, C07D 417/04, A61K 31/38, A61K 31/425

(21) Application number: **87113169.4**

(22) Date of filing: **09.09.87**

(54) **Heterocyclic amido derivatives of substituted benzoic acids and therapeutic compositions which contain them as active principle.**

(30) Priority: **23.09.86 IT 2178486**

(43) Date of publication of application:
**30.03.88 Bulletin 88/13**

(45) Publication of the grant of the patent:
**15.04.92 Bulletin 92/16**

(84) Designated Contracting States:
**AT BE CH DE ES FR GB GR IT LI LU NL SE**

(56) References cited:
**US-A- 2 401 522**
**US-A- 4 187 232**

**CHEMICAL ABSTRACTS, vol. 92, no. 25, 23rd June 1980, pages 619, 620, abstract no. 215368m, Columbus, Ohio, US; K. CLAUSEN et al.: "Studies on organophosphorus compounds. XXX. Synthesis of ethyl 2-(thioacylamino)-5-ethyl-3-thiophenecaroxylates and 2-substituted 6-ethylthieno[2,3-d][1,3]thiazine-4-thiones"**

(73) Proprietor: **VALEAS S.p.A. INDUSTRIA CHIMICA E FARMACEUTICA**
**via Vallisneri 10**
**I-20133 Milano(IT)**

(72) Inventor: **Bonifacio, Fausto**
**via Washington 98**
**I-20146 Milano(IT)**
Inventor: **Fano, Maurizio**
**Vicolo della Chiesa 15**
**I-20091 Bresso (MI)(IT)**
Inventor: **Trabella, Luciano**
**via Benzoni 35**
**I-27037 Pieve del Cairo (PV)(IT)**
Inventor: **Battigelli, Giandomenico**
**via Uruguay 7B**
**I-20159 Milano(IT)**
Inventor: **Montagna, Davide**
**via Angelo della Pergola 4**
**I-20159 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 87, no. 7, 15th August 1977, page 463, column 1, abstract no. 53186t, Columbus, Ohio, US; S. LEISTNER et al.: "Preparation of thiophenol[2,3-d]-, 6,7,8,9-tetrahydrobenzo[b]thiopheno[2,3-d]- and cyclohepteno[b]thiopheno[2,3-d]1,3-thiazines"**

**CHEMICAL ABSTRACTS, vol. 95, no. 25, 21st December 1981, page 511, column 2, abstract no. 220033q, Columbus, Ohio, US; K.E. NIELSEN et al.: "Phosphoramides. XIV. Phosphorus pentoxide and amine hydrochlorides as reagents in the synthesis of thieno[2,3-d]pyrimidin-4(3H)-ones"**

**CHEMICAL ABSTRACTS, vol. 80, no. 17, 29th April 1974, page 386, column 1, abstract no. 95642z, Columbus, Ohio, US; V.I. SHVEDOV et al.: "Functional thiophene derivatives. VI. Synthesis, azo coupling and aminomethylation of alpha-acylaminothiophenes"**

**CHEMICAL ABSTRACTS, vol. 79, no. 15, 15th October 1973, page 447, abstract no. 92269b, Columbus, Ohio, US; Patronato de Investigacion Cientifica y Tecnica "Juan de la Cerva" and Laboratorios Made S.A.: "Substituted 4-oxo-3-amino-3,4-dihydrothieno[3,2-d]-pyrimidine derivatives"**

**CHEMICAL ABSTRACTS, vol. 75, no. 11, 13th September 1971, page 457, column 1, abstract no. 76682t, Columbus, Ohio, US; L.M. WERBEL et al.: "Derivatives of 2-amino-5-nitrothiazole as potential schistosomicides"**

**CHEMICAL ABSTRACTS, vol. 85, no. 17, 25th October 1976, page 621, column 1, abstract no. 123697v, Columbus, Ohio, US; F. SAUTER et al.: "New derivatives of 2-(acylamino)thiophene-(and benzo[b]thiophene)-3-carboxylic acid and ([1]benzo-)thieno[2,3-d]pyrimidin-4(3H)-one"**

Inventor: **Bernareggi, Virgilio**
**v.le Piave 21**
**I-Cologno Monzese (MI)(IT)**

(74) Representative: **Gervasi, Gemma**
**NOTARBARTOLO & GERVASI Srl Viale Bianca Maria 33**
**I-20122 Milan(IT)**

## Description

This invention relates to heterocyclic amido derivatives of substituted benzoic acids of general formula (I):

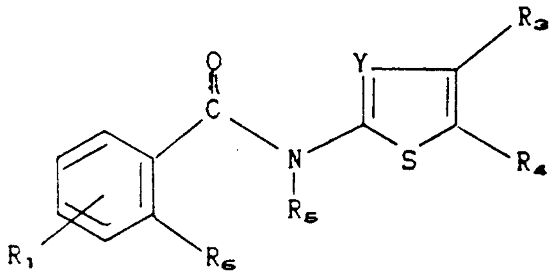

(I)

in which

$R_1$ is H; 3-$OCH_3$; 3-Cl; 4-Cl; 5-Cl; 3-$CH_3$; 5-$CH_3$; or 3-OH;

$R_3$ is H; -$CH_3$; -CO-COOH; -CO-$CO_2$-$C_2H_5$;

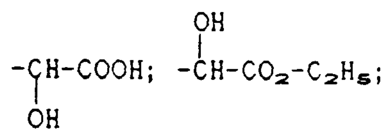

-$CH_2$-COOH; or -$CH_2$-$CO_2$-$CH_6$;

$R_4$ is H; a linear alkyl radical of 1-4 carbon atoms; -COOH; or -$CO_2C_2H_6$;

$R_5$ is H; or $R_5$ and $R_6$ together form (-CO-NH);

$R_6$ is H; -$NO_2$; -$NH_2$; -NH-CON; -NH-$COCH_3$; -NH-CO-CO$(CH_2)_n$-$CH_3$ where n is O, 1, 2 or 3; or $R_6$ and $R_5$ together form (NH-CO);

Y is N; or C-R in which R is $CO_2R_2$ and $R_2$ is H or an alkaline or alkaline-earth metal equivalent or a linear or branched alkyl radical containing 1-8 carbon atoms;

and their therapeutically acceptable salts, with the provisos defined in claim 1.

Said amido derivatives demonstrate surprisingly high activity as antiallergic agents, particularly when administered orally.

In the prophylactic treatment of allergic affections, and in particular in allergic bronchial asthma, the chosen medicament currently used in disodium cromoglycate. However this substance has the drawback of losing its pharmacological effectiveness when administered orally, as it is poorly absorbed at the gastrointestinal level and this widely limits its field of application. Antiallergic medicament for oral use are also known, such as tranilast [N-(3′,4′-dimethoxycinnamoyl) anthranilic acid], however these are products without satisfactory antiallergic activity.

The object of the present invention is to provide products which, when administered orally, are easily absorbed at the digestive tube level and exert a powerful antiallergic action.

This object is attained by the heterocyclic amido derivatives of substituted benzoic acids of general formula (I) according to the present invention.

To prepare the compounds of formula (I), the starting substances used are o-nitrobenzoic or p-nitrobenzoic acids, for example an o-nitrobenzoic acid of formula (II)

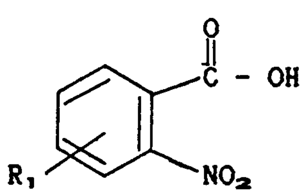

(II)

in which R, has the aforesaid meaning, and 2-aminothiophene derivatives of general formula (III)

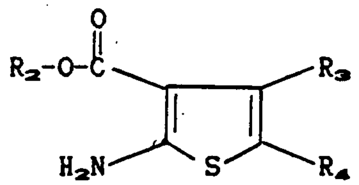

(III)

or 2-aminothiazole derivatives of general formula (IV)

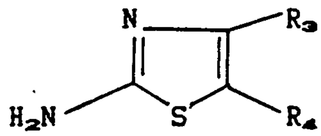

(IV)

in which $R_2$, $R_3$ and $R_4$ have the aforesaid meanings.

In a preferred method, the o-nitrobenzoic acid (II) is firstly converted into the corresponding chloride (V)

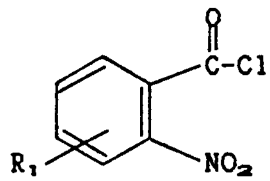

(V)

by treatment with conventional chlorinating agents such as $SOCl_2$, $POCl_3$, $PCl_5$ or oxalyl chloride under suitable conditions.

The next step is to condense the acid chloride (V) with the 2-aminothiophene derivative (III) or with the 2-aminothiazole derivative (IV), according to the type of product to be obtained

The reaction of this step is conducted in a reaction medium consisting of an aprotic organic solvent in the presence of a basic substance.

Examples of solvents which can be used are $CHCl_3$, $CH_2Cl_2$, acetone, benzene, toluene, THF, dioxane and DMF, the preferred solvent being $CH_2Cl_2$ or $CHCl_3$.

Examples of basic substances which can be used are tertiary organic bases such as $Me_3N$, $Et_3N$, pyridine and alkaline bases such as $K_2CO_3$ or $Na_2CO_3$, the preferred base being triethylamine or potassium carbonate.

The molar ratio of (V) to (III) or of (V) to (IV) used in the reaction lies between 1 and 2.5, and preferably between 1 and 1.2.

The reaction is conducted at a temperature of between 40 and 160 ° C, and preferably between 40 and 70 ° C, under agitation for a time of between 1 and 7 hours.

A modification to the aforesaid condensation reaction consists of directly treating the o-nitrobenzoic acid of formula (II) with the 2-aminothiophene derivative (III) or with the 2-aminothiazole derivative (IV).

This reaction is conducted in a reaction medium consisting of an organic solvent in the presence of a condensing agent.

Solvents such as THF, benzene, dioxane or EtOH can be used as the reaction medium, the preference being for THF.

Dicyclohexylcarbodiimide, ethoxyacetylene, diethylcyanamide or triphenylphosphite + imidazole can be used as condensing agents, the preferred agent being dicyclohexylcarbodiimide.

The reaction is conducted at a temperature of between 0 and 60 ° C and preferably between 0 and 5 ° C.

The molar ratio of compound (II) to compound (III) or of compound (II) to compound (IV) is between 0.5 and 1 and preferably between 0.5 and 0.67.

The aforesaid synthesis processes lead to the formation of amides characterised by the presence of the nitro group in the aromatic ring, they being of general formula (VI)

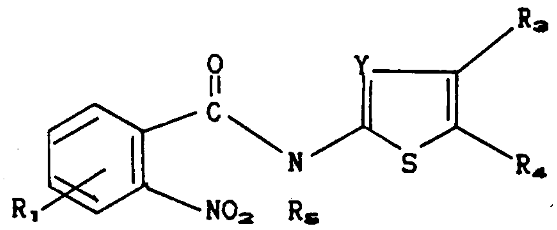

(VI)

in which $R_1$, $R_3$, $R_4$, $R_5$ and Y have the aforesaid meanings.

The nitro group can then be reduced to an amino group to give products of general formula (VII)

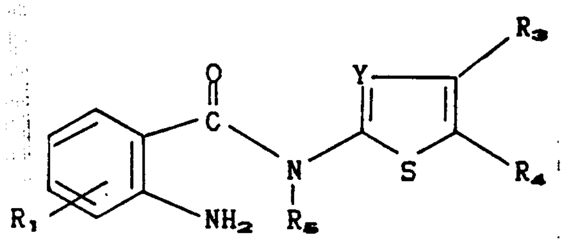

(VII)

The reduction can be conveniently conducted by treatment with hydrogen under pressure in the presence of a suitable catalyst, such as 10% Pd on C.

The reduction is generally carried out under pressure. If $R_3$ is $CO\text{-}CO_2\text{-}C_2H_5$, the reduction is carried out at atmospheric pressure.

The amino group of the compounds of general formula (VII) can be functionalised with acyl groups to provide for example N-acetyl derivatives by reaction with acetic anhydride in acetic acid, or with N-oxamic esters by reaction with alkyl oxalyl chlorides. These are prepared by the method of John B. Wright, Charles M. Hall and Herbert G. Johnson (J. Med. Chem. 1978, vol 21, 930-935).

By treatment with $COCl_2$ in toluene, the compounds of general formula (VII) can be converted into tricyclic compounds of general formula (VIII)

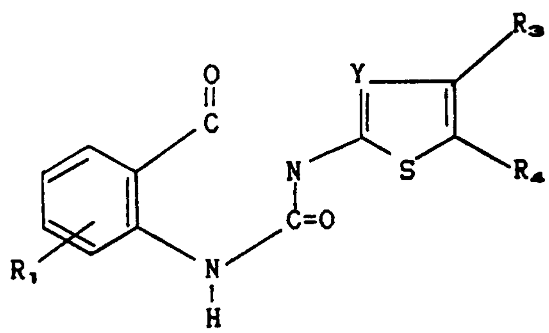

(VIII)

in which $R_1$, $R_3$, $R_4$ and Y have the aforesaid meanings. The reaction is conducted at a temperature between ambient and the reflux temperature.

On the basis of the aforegoing description, all possible modifications, which also lie within the scope of the invention, are immediately apparent to experts of the art.

In order to ilustrate some preferred embodiments of the process according to the present invention, examples are given hereinafter of the method for preparing the new compounds.

The invention is illustrated but not limited by sold examples.

Table 1 lists the chemical characteristics of 38 compounds according to the invention, and the yields obtained in their preparation.

EXAMPLE 1

3-THIOPHENE CARBOXYLIC ACID (2-AMINO-5-METHYL) ETHYL ESTER

46.96 g of triethylamine (0.465 moles) are added under energetic agitation to a mixture of 97.4 g of ethylcyanoacetate (0.862 moles), 27.58 g of sulphur (0.862 moles) and 107 ml of dimethylformamide.

50 g of propionaldehyde (0.862 moles) are dripped into this suspension, while maintaining the temperature at 50°C, and when the addition is complete it is allowed to reach ambient temperature and then kept under agitation for 2 hours. An oil forms which is treated with ether and water. The ether phase is separated, dried with sodium sulphate and evaporated to dryness to obtain an oil which is distilled under vacuum (boiling point 161-162°C at 13 mmHg).

88 g of 3-thiophene carboxylic acid (2-amino-5-methyl) ethyl ester are obtained. (Yield 55%. U.V. purity 99.8%).

| Analysis for $C_8H_{11}NO_2S$ | % calculated | % found |
|---|---|---|
| C | 51.86 | 52.08 |
| H | 5.98 | 5.91 |
| N | 7.56 | 7.64 |

Using a procedure analogous to the aforesaid example, the following compounds are prepared:
3-THIOPHENE CARBOXYLIC ACID, (2-AMINO-5-METHYL) n.PROPYL ESTER
3-THIOPHENE CARBOXYLIC ACID, (2-AMINO-5-ETHYL) ETHYL ESTER
3-THIOPHEHE CARBOXYLIC ACID, (2-AMIHO-5-n.PROPYL) ISOPROPYL ESTER
3-THIOPHENE CARBOXYLIC ACID, (2-AMINO-5-n.BUTYL) n.PENTYL ESTER
3-THIOPHENE CARBOXYLIC ACID, (2-AMINO-5-METHYL) tert.BUTYL ESTER
3-THIOPHENE CARBOXYLIC ACID, (2-AMINO-5-ETHYL) n.OCTYL ESTER.

EXAMPLE 2

3-THIOPHENE CARBOXYLIC ACID, 2[(2-AMINO-3-METHOXY BENZOYL)AMINO]-5-METHYL-ETHYL ESTER (No. 7 of Table 1)

3-methoxy-2-nitro benzoyl chloride:

100 g of 3-methoxy-2-nitrobenzoic acid (0.507 moles) are mixed in a dry apparatus under a stream of nitrogen with 602.8 g of freshly distilled thionylchloride (5.07 moles). The mixture is heated under reflux for 7 hours. It is cooled, the excess thionylchloride distilled off with a water pump, the solid residue taken up in anhydrous n-hexane, filtered off through a Buchner funnel and then dried to obtain 103 g of 3-methoxy-2-nitro benzoylchloride. (Yield 94.2%, M.P. 84-86°C, $Cl^-$ purity 98.8%).

| Analysis for $C_8H_6NO_4Cl$ | % calculated | % found |
|---|---|---|
| C | 44.56 | 44.44 |
| H | 2.80 | 2.88 |
| N | 6.49 | 6.38 |

3-thiophene carboxylic acid-2-[(2-nitro-3-methoxy benzoyl)amino]-5-methyl ethyl ester (No. 1 of Table 1):

METHOD A

20.7 g of 3-thiophene carboxylic acid-2-amino-5-methyl ethyl ester (0.112 moles) dissolved in 250 ml of anhydrous methylene chloride and 29.2 g of triethylamine (0.289 moles) are introduced into a dry apparatus. 29 g of 3-methoxy-2-nitro benzoyl chloride (O. 134 moles ) dissolved in 343 ml of anhydrous methylene chloride are dripped into this solution.

The mixture is heated under reflux for 7 hours, after which the solvent is evaporated and the solid obtained is left in 690 ml of ethanol under agitation for 1 hour.

The solid residue is filtered off through a Buchner funnel and dried. 32.4 g of 3-thiophene carboxylic

acid-2-[(2-nitro-3-methoxy benzoyl)amino]-5-methyl ethyl ester are obtained. (Yield 79.5%, M.P. 168-170 ° C, U.V. purity 100%).

| Analysis for $C_{16}H_{16}N_2O_6S$ | % calculated | % found |
|---|---|---|
| C | 52.73 | 52.68 |
| H | 4.42 | 4.51 |
| N | 7.68 | 7.73 |

METHOD B

3.178 g of potassium carbonate (0.023 moles) and 4.959 g of 3-methoxy-2-nitro benzoyl chloride (0.023 moles) dissolved in 50 ml of anhydrous methylene chloride are introduced into the dry apparatus. The temperature is reduced to 0 ° C, after which 4.255 g of 3-thiophene carboxylic acid-2-amino-5-methyl ethyl ester (0.023 moles) dissolved in 70 ml of anhydrous methylene chloride are dripped in.

When this addition is terminated, the temperature is allowed to rise to 25 ° C after which the mixture is heated under reflux for 2 hours.

The reaction mixture is filtered through a Buchner funnel, and the inorganic salt is washed with methylene chloride. The phases are separated, the organic phase being dried with sodium sulphate and then evaporated to dryness.

A solid is obtained which is crystallised from 75 mi of ethyl acetate.

5.693 g of 3-thiophene carboxylic acid-2-[(2-nitro-3-methoxy benzoyl)amino]-5-methyl ethyl ester are obtained. (Yield 68%, M.P. 171-172 ° C, U.V. purity 99.8%).

| Analysis for $C_{16}H_{16}N_2O_6S$ | % calculated | % found |
|---|---|---|
| C | 52.73 | 52.80 |
| H | 4.42 | 4.43 |
| N | 7.68 | 7.83 |

METHOD C

10 g of 3-methoxy-2-nitro benzoic acid (0.05 moles) dissolved in 60 ml of tetrahydrofuran, 9.25 g of 3-thiophene carboxylic acid-2-amino-5-methyl ethyl ester (0.05 moles) dissolved in 90 ml of tetrahydrofuran and 0.5 g of N,N-dimethylaminopyridine (0.004 moles) as catalyst are introduced into a flask maintained under a nitrogen atmosphere. The tetrahydrofuran had previously been dried by distillation over lithium aluminium hydride.

The mixture temperature is reduced to 0 ° C and 10.68 g of dicyclohexylcarbodiimide (0.0518 moles) are dripped in.

The mixture temperature is allowed to reach ambient, and this is maintained for 2 hours.

The precipitate formed is filtered off and the inorganic phase evaporated to dryness.

The solid obtained is crystallised from 200 ml of ethyl acetate.

After filtration and drying, 8.19 g of 3-thiophene carboxylic acid-2-[(2-nitro-3-methoxy benzoyl)amino]-5-methyl ethyl ester are obtained. (Yield 45%, M.P. 170-171 ° C, U.V. purity 98 9%).

| Analysis for $C_{16}H_{16}N_2O_6S$ | % calculated | % found |
|---|---|---|
| C | 52.73 | 52.60 |
| H | 4.42 | 4.38 |
| N | 7.68 | 7.59 |

3-thiophene carboxylic acid-2-[(2-amino-3-methoxy benzoyl)amino]-5-methyl ethyl ester:

51 g of 3-thiophene carboxylic acid-2-[(2-nitro-3-methoxy benzoyl)amino]-5-methyl ethyl ester (0.140 moles) dissolved in 670 ml of tetrahydrofuran, and containing 14.84 g of 10% palladium on carbon, are introduced into a Parr apparatus for hydrogenation under pressure (51 psi = 3.47 atm).

On termination of hydrogen consumption, the catalyst is removed by filtration and the mixture evaporated to dryness.

The solid obtained is crystallised from 750 ml of ethyl acetate.

The crystallized product is filtered through a Buchner funnel and dried. 34.382 g of 3-thiophene carboxylic acid-2-[(2-amino-3-methoxy benzoyl)amino]-5-methyl ethyl ester are obtained. (Yield 73.5%, M.P. 188-189 ° C, U.V. purity 99.8%).

| Analysis for $C_{16}H_{18}N_2O_4S$ | % calculated | % found |
|---|---|---|
| C | 57.46 | 57.29 |
| H | 5.42 | 5.43 |
| N | 8.37 | 8.38 |

Using a procedure analogous to the aforesaid example, the following compounds are prepared:

3-THIOPHENE CARBOXYLIC ACID, 2[(2-AMINO-3-METHOXY-BENZOYL)AMINO]-5-METHYL-n.PROPYL ESTER

3-THIOPHENE CARBOXYLIC ACID, 2[(2-AMINO-3-METHOXY-BENZOYL)AMINO]-5-METHYL-tert.BUTYL ESTER

3-THIOPHENE CARBOXYLIC ACID, 2[(2-AMINO-3-METHOXY-BENZOYL)AMINO]-5-ETHYL-ETHYL ESTER

3-THIOPHENE CARBOXYLIC ACID, 2[(2-AMINO-3-METHOXY-BENZOYL)AMINO]-5-n.PROPYL-ISOPROPYL ESTER

3-THIOPHENE CARBOXYLIC ACID, 2[(2-AMINO-3-METHOXY-BENZOYL)AMINO]-5-n.BUTYL-n.PENTYL ESTER

3-THIOPHENE CARBOXYLIC ACID, 2[(2-AMINO-3-METHOXY-BENZOYL)AMINO]-5-ETHYL-n.OCTYL ESTER.

EXAMPLE 3

3-THIOPHEHE CARBOXYLIC ACID, 2-[(2-AMINO-5-CHLORO BENZOYL)AMINO]-5-METHYL-ETHYL ESTER (No. 10 of Table 1)

5-chloro-2-nitro benzoyl chloride:

55g of 5-chloro-2-nitro benzoic acid (0.273 moles) and 324.79 g of thionyl chloride (2.73 moles) are reacted under reflux for 4 hours in the dry apparatus. On termination of the reaction, the excess thionyl chloride is distilled off and the residue is distilled with a high vacuum pump (M.P. 113-114 ° C at 0.3 mmHg). 51.128 g of 5-chloro-2-nitro benzoyl chloride are obtained. (Yield 85%, U.V. purity 98.4%).

| Analysis for $C_7H_3NO_3Cl_2$ | % calculated | % found |
|---|---|---|
| C | 38.21 | 38.30 |
| H | 1.37 | 1.32 |
| N | 6.36 | 6.29 |

3-thiophene carboxylic acid-2-[(2-nitro-5-chloro benzoyl)amino]-5-methyl ethyl ester (No. 4 of Table 1):

12.57 g of potassium carbonate (0.091 moles) and 20 g of 5-chloro-2-nitro benzoyl chloride (0.091 moles) dissolved in 52 ml of anhydrous methylene chloride are introduced into a previously dried flask in a stream of nitrogen.

16.835 g of 3-thiophene carboxylic acid-2-amino-5-methyl ethyl ester (0.091 moles) dissolved in 130 ml of anhydrous methylene chloride are dripped in at 0 °C.

On termination of the addition, the mixture temperature is allowed to rise to ambient, after which it is boiled for 2 hours.

The reaction mixture is filtered through a Buchner funnel to eliminate the insoluble solid, and the organic phase is washed with water. This latter is then dried with sodium sulphate and evaporated to dryness.

The solid obtained is crystallised from 75 ml of ethyl acetate, the precipitate being filtered off through a Buchner funnel and dried.

22.8 g of 3-thiophene carboxylic acid-2-[(2-nitro-5-chloro benzoyl)amino]-5-methyl ethyl ester are obtained. (Yield 68%, M.P. 150-152 °C).

| Analysis for $C_{15}H_{13}N_2O_6ClS$ | % calculated | % found |
|---|---|---|
| C | 48.84 | 49.02 |
| H | 3.55 | 3.58 |
| N | 7.59 | 7.62 |

3-thiophene carboxylic acid, 2-[(2-amino-5-chloro benzoyl)amino]-5-methyl ethyl ester:

10.720 g of 3-thiophene carboxylic acid, 2-[(2-nitro-5-chloro benzoyl)amino]-5-methyl ethyl ester (0.029 moles) dissolved in 90 ml of anhydrous tetrahydrofuran are introduced into a Parr hydrogenator, 2.5 g of 5% palladium oxide are added, and the mixture treated with hydrogen at a pressure of 27 psi (1.84 atm).

On termination of the hydrogenation, the catalyst is filtered off through celite and the organic phase evaporated.

The solid residue is chromatographed in a silica gel column using N-hexane/acetone 7:3 as eluent.

The fractions containing the pure product are pooled and evaporated to dryness.

5.5 g of 3-thiophene carboxylic acid, 2-[(2-amino-5-chloro benzoyl)amino]-5-methyl ethyl ester are obtained. (Yield 56%, M.P. 148-150 °C).

| Analysis for $C_{15}H_{15}N_2O_2ClS$ | % calculated | % found |
|---|---|---|
| C | 53.17 | 53.06 |
| H | 4.46 | 4.48 |
| N | 8.27 | 8.32 |

Using a procedure analogous to the aforesaid example, the following compounds are prepared:

3-THIOPHENE CARBOXYLIC ACID, 2-[(2-AMINO-5-CHLOROBENZOYL)AMINO]-5-METHYL-n.PROPYL ESTER

3-THIOPHENE CARBOXYLIC ACID, 2-[(2-AMINO-5-CHLOROBENZOYL)AMINO]-5-METHYL-tert.BUTYL ESTER

3-THIOPHENE CARBOXYLIC ACID, 2-[(2-AMINO-5-CHLOROBENZOYL)AMINO]-5-ETHYL-ETHYL ESTER

3-THIOPHENE CARBOXYLIC ACID, 2-[(2-AMINO-4-CHLOROBENZOYL)AMINO]-5-n.BUTYL-n.PENTYL ESTER

3-THIOPHENE CARBOXYLIC ACID, 2-[(2-AMINO-3-CHLOROBENZOYL)AMINO]-5-ETHYL-ETHYL ESTER

3-THIOPHENE CARBOXYLIC ACID, 2-[(2-AMINO-4-CHLOROBENZOYL)AMINO]-5-ETHYL-n.OCTYL ESTER

3-THIOPHENE CARBOXYLIC ACID, 2-[(2-AMINO-3-CHLOROBENZOYL)AMINO]-5-n.PROPYL-ISOPROPYL ESTER.

EXAMPLE 4

3-THIOPHENE CARBOXYLIC-ACID-(2-AMINO-5-n.BUTYL)-METHYL ESTER

27.29 g of triethylamine (0.27 moles) are added to a mixture of 49.5 g of ethyl cyanoacetate (0.5

moles), 16 g of sulphur (0.5 moles) and 62.5 ml of dimethylformamide under energetic agitation.

50 g of hexanal (0.5 moles) are dripped into this suspension while maintaining the temperature at 50 ° C, after which the temperature is allowed to fall to ambient at which the mixture is maintained under agitation for 2 hours.

An oil separates and is treated with ether and water. The ether phase is separated, dried over sodium sulphate and evaporated to dryness to obtain an oil which is distilled under vacuum. M.P. 222-223 ° C at 25 mmHg.

54.32 g of 3-thiophene carboxylic acid-(2-amino-5-methyl)-ethyl ester are obtained. (Yield 51%, M.P. 64-66 ° C).

| Analysis for $C_{10}H_{15}NO_2S$ | % calculated | % found |
|---|---|---|
| C | 56.30 | 56.09 |
| H | 7.08 | 7.15 |
| N | 6.56 | 6.62 |

EXAMPLE 5

3-THIOPHENE CARBOXYLIC ACID, 2-[(2-AMINO-5-METHYL BENZOYL)AMINO]-5-n.BUTYL-METHYL ESTER (No. 14 of Table 1)

5-methyl-2-nitro benzoyl chloride:

5 g of 5-methyl-2-nitro benzoic acid (0.027 moles) are finely ground in a mortar with 2.4 g of sodium bicarbonate (0.028 moles). The resultant powder is dissolved in water under agitation and slight heating. The water is evaporated under vacuum to obtain 4.76 g of 5-methyl-2-nitro benzoic acid, sodium salt. This quantity (0.0234 moles), suspended in 110 ml of anhydrous tetrahydrofuran, is reacted in an anhydrous environment at 0 ° C with 7.4 g of oxalyl chloride (0.054 moles). On termination of the reaction, both the excess oxalyl chloride and solvent are removed. The solid is taken up in anhydrous n-hexane, filtered off through a Buchner funnel and dried.

4.25 g of 5-methyl-2-nitro benzoyl chloride are obtained. (Yield 78.8%, M.P. 32-33 ° C).

| Analysis for $C_8H_6NO_3Cl$ | % calculated | % found |
|---|---|---|
| C | 48.13 | 48.21 |
| H | 3.03 | 2.91 |
| N | 7.02 | 6.98 |

3-thiophene carboxylic acid 2-[(2-nitro-5-methyl benzoyl)amino]-5-n.butyl-methyl ester (No. 13 of Table 1):

20 g of 3-thiophene carboxylic acid-(2-amino-5-n.butyl)-methyl ester (0.094 moles) dissolved in 230 ml of anhydrous methylene chloride and 24.4 g of triethylamine (0.241 moles) are introduced into a dry apparatus maintained under a stream of nitrogen. 22.5 g of 5-methyl-2-nitro benzoyl chloride (0.112 moles) are rapidly dripped into this solution, and the mixture then heated under reflux for 7 hours. On termination of the reaction, the solvent is evaporated and the solid residue agitated with 700 ml of ethanol for one hour. It is filtered off through a Buchner funnel and dried to obtain 24.44 g of 3-thiophene carboxylic acid, 2-[(2-nitro-5-methyl benzoyl)amino]-5-n.butyl-ethyl ester. (Yield 69%, M.P. 120-122 ° C).

| Analysis for $C_{18}H_{20}N_2O_5S$ | % calculated | % found |
|---|---|---|
| C | 57.43 | 57.39 |
| H | 5.35 | 5.34 |
| N | 7.44 | 7.50 |

3-thiophene carboxylic acid, 2-[(2-amino-5-methyl benzoyl)amino]-5-n.butyl-methyl ester:

10 g of 3-thiophene carboxylic acid, 2-[(2-nitro-5-methyl benzoyl)amino]-5-n.butyl-methyl ester (0.0266 moles) are dissolved in 400 ml of anhydrous tetrahydrofuran, 2.8 g of 10% palladium on carbon are added and the mixture introduced into a Parr apparatus for hydrogenation under pressure (51 psi = 3.47 atm). On termination of the reaction, the catalyst is filtered off through celite and the solvent evaporated. The solid residue is crystallized from 100 ml of ethyl acetate. The precipitate is filtered off through a Buchner funnel and then dried to obtain 5.06 g of 3-thiophene carboxylic acid, 2-[(2-amino-5-methyl benzoyl)amino]-5-n.butyl-methyl ester. (Yield 55%, M.P. 152-154° C).

| Analysis for $C_{18}H_{22}N_2O_3S$ | % calculated | % found |
|---|---|---|
| C | 62.40 | 61.90 |
| H | 6.40 | 6.28 |
| N | 8.08 | 8.10 |

Using a procedure analogous to the aforesaid example, the following compounds are prepared:
3-THIOPHENE CARBOXYLIC ACID, 2-[(2-AMINO-5-METHYL BENZOYL)AMINO]-5-METHYL-n.PROPYL ESTER
3-THIOPHENE CARBOXYLIC ACID, 2-[(2-AMINO-5-METHYL BENZOYL)AMINO]-5-METHYL-tert.BUTYL ESTER
3-THIOPHENE CARBOXYLIC ACID, 2-[2-AMINO-5-METHYL BENZOYL)AMINO]-5-n.BUTYL-n.PENTYL ESTER
3-THIOPHENE CARBOXYLIC ACID, 2[(AMINO-3-METHYL BENZOYL)AMINO]-5-ETHYL-ETHYL ESTER
3-THIOPHENE CARBOXYLIC ACID, 2-[(2-AMINO-3-METHYL BENZOYL)AMINO]-5-n.PROPYL-ISOPROPYL ESTER
3-THIOPHENE CARBOXYLIC ACID, 2-[(2-AMINO-3-METHYL BENZOYL)AMINO]-5-ETHYL-n.OCTYL ESTER.

EXAMPLE 6

5-THIAZOLE CARBOXYLIC ACID, 2-[(2-AMINO-3-METHOXY BENZOYL)AMINO] 4-METHYL ESTER (No. 23 of Table 1)

5-thiazole carboxylic acid, 2-[(2-nitro-3-methoxy benzoyl)amino] 4-methyl ethyl ester (No. 22 of Table 1):

5.174 g of 3-methoxy-2-nitro benzoyl chloride (prepared in accordance with Example 2) (0,024 moles) dissolved in 55 ml of anhydrous acetone and 3.317 g of anhydrous potassium carbonate (0.024 moles) are introduced into a dry apparatus and maintained under a stream of nitrogen.

The mixture temperature is lowered to 0° C and a solution of 4.464 g of 5-thiazole carboxylic acid, 2-amino-4-methyl, ethyl ester (0.024 moles) in 120 ml of anhydrous acetone is dripped in, during which the temperature is maintained at 0° C.

On termination of this addition, the mixture temperature is allowed to rise to ambient after which it is heated under reflux for 3 hours.

On termination of the heating, the solvent is evaporated and the solide residue is chromatographed in a silica gel column using chloroform/methanol 9.5:0.5 as eluent.

The fractions containing the product are pooled and evaporated to dryness.

The solid residue is taken up in 80 ml of ethyl ether, left under agitation for one hour, filtered through a

Buchner funnel and dried.

5.7 g of 5-thiazole carboxylic acid, 2-[(2-nitro-3-methoxy benzoyl)amino]-4-methyl, ethyl ester are obtained. (Yield 65%, M.P. 260-261 °C).

| Analysis for $C_{15}H_{15}N_3O_6S$ | % calculated | % found |
|---|---|---|
| C | 49.31 | 49.21 |
| H | 4.14 | 4.08 |
| N | 11.50 | 11.36 |

5-thiazole carboxylic acid, 2-[(2-amino-3-methoxy benzoyl)amino]-4-methyl, ethyl ester:

2 g of 5-thiazole carboxylic acid, 2-[(2-nitro-3-methoxy benzoyl)amino]-4-methyl, ethyl ester (0.0054 moles) dissolved in 200 ml of tetrahydrofuran are introduced into a Parr apparatus for hydrogenation under pressure, 1 g of 10% palladium on carbon is added to the mixture and hydrogenation is carried out while maintaining the hydrogen pressure around 51 psi = 3.47 atm.

On termination of the reaction, the catalyst is filtered off through celite and the solvent evaporated. The solid residue is crystallised from 50 ml of ethanol, filtered off through a Buchner funnel and dried.

1.190 g of 5-thiazole carboxylic acid, 2-[(2-amino-3-methoxy benzoyl)amino]-4-methyl, ethyl ester are obtained. (Yield 64. 9%, M.P. 176-177 °C).

| Analysis for $C_{15}H_{17}N_3O_4S$ | % calculated | % found |
|---|---|---|
| C | 53.71 | 53.57 |
| H | 5.11 | 4.99 |
| N | 12.53 | 12.61 |

Using a procedure analogous to the aforesaid example, the following compounds are prepared:
5-THIAZOLE CARBOXYLIC ACID, 2-[(2-AMINO-3-CHLORO BENZOYL)AMINO]-4-METHYL, ETHYL ESTER
5-THIAZOLE CARBOXYLIC ACID, 2-[(2-AMINO-5-CHLORO BENZOYL)AMINO]-4-METHYL, ETHYL ESTER
5-THIAZOLE CARBOXYLIC ACID, 2-[(2-AMINO-4-CHLORO BENZOYL)AMINO]-4-METHYL, ETHYL ESTER
5-THIAZOLE CARBOXYLIC ACID, 2-[(2-AMINO-3-METHYL BENZOYL)AMINO]-4-METHYL, ETHYL ESTER
5-THIAZOLE CARBOXYLIC ACID, 2-[(2-AMINO-5-METHYL BENZOYL)AMINO]-4-METHYL, ETHYL ESTER.

EXAMPLE 7

4-THIAZOLE ACETIC ACID, 2-[(2-AMINO-3-METHOXY BENZOYL)AMINO], ETHYL ESTER (No. 25 of Table 1)

4-thiazole acetic acid, 2-[(2-nitro-3-methoxy benzoyl)amino], ethyl ester (No. 24 of Table 1):

10 g of 3-methoxy-2-nitro benzoyl chloride (prepared in accordance with Example 2) (0.046 moles) dissolved in 160 ml of anhydrous methylene chloride and 6.41 g of anhydrous potassium carbonate (0.046 moles) are introduced into a dry apparatus and maintained under a nitrogen stream.

The mixture temperature is reduced to 0 °C and a solution of 8.627 g of 4-thiazole acetic acid-2-amino, ethyl ester (0.046 moles) in 150 ml of anhydrous methylene chloride is dripped in. During the addition the temperature is kept around 0 °C.

On termination of the addition the temperature of the reaction mixture is allowed to rise to ambient, after which it is heated under reflux for 3 hours.

On termination of heating, it is cooled and 300 ml of water added. The organic phase is separated, dried with sodium sulphate and evaporated to dryness.

The solid residue is chromatographed in a silica gel column using chloroform/acetone 6:4 as eluent.

The fractions containing the pure product are pooled and evaporated to dryness.

The solid obtained is crystallised from 150 ml of ethanol.

10.336 g of 4-thiazole acetic acid, 2-[(2-nitro-3-methoxy benzoyl)amino], ethyl ester are obtained. (Yield 61%, M.P. 165-166°C).

| Analysis for $C_{15}H_{15}N_3O_6$5 | % calculated | % found |
|---|---|---|
| C | 49.31 | 49.51 |
| H | 4.14 | 4.08 |
| N | 11.50 | 11.38 |

4-thiazole acetic acid, 2-[(2-amino-3-methoxy benzoyl)amino], ethyl ester:

3 g of 4-thiazole acetic acid, 2-[(2-nitro-3-methoxy benzoyl)-2-amino], ethyl ester (0.0082 moles) dissolved in 300 ml of methanol are introduced into a Parr apparatus for hydrogenation under pressure, 1 g of 10% palladium on carbon is added and the hydrogenation carried out maintaining the hydrogen pressure around 51 psi = 3.47 atm.

On termination of the reaction, the catalyst is filtered off through celite and the solvent evaporated.

The oily residue is dissolved at 50-60°C in 25 ml of ethanol. It is cooled and at ambient temperature a solid precipitates which is filtered off and dried.

1.676 g of 4-thiazole acetic acid, 2-[(2-amino-3-methoxy benzoyl)2-amino], ethyl ester are obtained. (Yield 61%, M.P. 133-135°C).

| Analysis for $C_{15}H_{17}N_3O_4S$ | % calculated | % found |
|---|---|---|
| C | 53.71 | 53.42 |
| H | 5.11 | 5.21 |
| N | 12.53 | 12.40 |

Using a procedure analogous to the aforesaid example, the following compounds are prepared:
4-THIAZOLE ACETIC ACID, 2-[(2-AMINO-3-CHLORO BENZOYL)AMINO], ETHYL ESTER
4-THIAZOLE ACETIC ACID, 2-[(2-AMINO-5-CHLORO BENZOYL)AMINO], ETHYL ESTER
4-THIAZOLE ACETIC ACID, 2-[(2-AMINO-4-CHLORO BENZOYL)AMINO], ETHYL ESTER
4-THIAZOLE ACETIC ACID, 2-[(2-AMINO-3-METHYL BENZOYL)AMINO], ETHYL ESTER
4-THIAZOLE ACETIC ACID, 2-[(2-AMINO-5-METHYL BENZOYL)AMINO], ETHYL ESTER.

EXAMPLE 8

4-THIAZOLE GLYOXYLIC ACID, 2-[(2-AMINO-3-METHOXY)BENZOYL)AMINO] ETHYL ESTER (No. 27 of Table 1)

4-thiazole glyoxylic acid, 2-[(2-nitro-3-methoxy benzoyl)amino]-ethyl ester (No. 26 of Table 1):

5.13 g of 3-methoxy-2-nitro benzoyl chloride (prepared in accordance with Example 2) (0.0238 moles) dissolved in 110 ml of tetrahydrofuran and 3.3 g of anhydrous potassium carbonate (0.0239 moles) are introduced into a dry apparatus and maintained under a nitrogen stream.

The mixture temperature is reduced to 0°C, and a solution of 4.78 g of 4-thiazole glyoxylic acid, 2-amino, ethyl ester (0.0239 moles) in 120 ml of anhydrous tetrahydrofuran is dripped in. During this addition the temperature is maintained at 0°C.

On termination of the addition, the mixture temperature is increased to ambient after which it is heated under reflux for 9 hours.

On termination of heating, the mixture is cooled and 300 ml of water added. The mixture is extracted with 300 ml of chloroform and the phases separated, the organic phase being dried and evaporated. A solid is obtained which is crystallised from 130 ml of acetone. The precipitate is filtered off through a Buchner funnel and dried. 5.68 g of 4-thiazole glyoxylic acid, 2-[(2-nitro-3-methoxy benzoyl)amino]-ethyl ester are obtained. (Yield 63%, M.P. 240-242°C).

| Analysis for $C_{15}H_{13}N_3O_7S$ | % calculated | % found |
|---|---|---|
| C | 47.49 | 47.26 |
| H | 3.45 | 3.35 |
| N | 11.07 | 11.01 |

4-thiazole glyoxylic acid, 2-[(2-amino-3-methoxy benzoyl)amino]-ethyl ester:

5 g of 4-thiazole glyoxylic acid, 2-[(2-nitro-3-methoxy benzoyl) amino]-ethyl ester (0.0132 moles) dissolved in 100 ml of tetrahydrofuran are introduced into an apparatus for hydrogenation at atmospheric pressure, 1.4 g of 10% palladium on carbon is added to the mixture and hydrogenation is carried out.

On termination of the reaction, the catalyst is filtered off through celite and the solvent evaporated. The solid residue is crystallised from 70 ml of ethanol, filtered off through a Buchner funnel and dried.

2.63 g of 4-thiazole glyoxylic acid, 2-[(2-amino-3-methoxy benzoyl)amino]-ethyl ester are obtained. (Yield 57%, M.P. 165-166° C).

| Analysis for $C_{15}H_{15}N_3O_5S$ | % calculated | % found |
|---|---|---|
| C | 51.56 | 51.49 |
| H | 4.32 | 4.33 |
| N | 12.02 | 11.98 |

Using a procedure analogous to the aforesaid example, the following compounds are prepared:
4-THIAZOLE GLYOXYLIC ACID, 2-[(2-AMINO-3-CHLORO BENZOYL)AMINO]-ETHYL ESTER
4-THIAZOLE GLYOXYLIC ACID, 2-[(2-AMINO-4-CHLORO BENZOYL)AMINO]-ETHYL ESTER
4-THIAZOLE GLYOXYLIC ACID, 2-[(2-AMINO-5-CHLORO BENZOYL)AMINO]-ETHYL ESTER
4-THIAZOLE GLYOXYLIC ACID, 2-[(2-AMINO-3-METHYL BENZOYL)AMINO]-ETHYL ESTER
4-THIAZOLE GLYOXYLIC ACID, 2-[(2-AMINO-5-METHYL BENZOYL)AMINO]-ETHYL ESTER
4-THIAZOLE GLYOXYLIC ACID, 2-[(2-AMINO-3-METHOXY BENZOYL)AMINO]-5-ETHYL ESTER.

EXAMPLE 9

3-THIOPHENE CARBOXYLIC ACID, 2-[(2-ACETYL AMINO-3-METHOXY BENZOYL)AMINO]-5-METHYL, ETHYL ESTER (No. 15 of Table 1)

A suspension composed of 17.8 g of 3-thiophene carboxylic acid 2-[(2-amino-3-methoxy benzoyl)-amino]-5-methyl, ethyl ester (0.053 moles) (prepared in accordance with Example 2), 85 ml of acetic acid and 16.3 g of acetic anhydride (0.16 moles) is heated to 80° C for half hour. The hot reaction mixture is dripped into 1200 ml of water and ice. The solid which precipitates is filtered off through a Buchner funnel and crystallised from 290 ml of ethyl acetate. After drying the solid, 12.086 g of 3-thiophene carboxylic acid, 2-[(2-acetyl amino-3-methoxy benzoyl)amino]-5-methyl, ethyl ester are obtained. (Yield 60.6%, M.P. 194-196° C, U.V. purity 100%).

| Analysis for $C_{18}H_{20}N_2O_5S$ | % calculated | % found |
|---|---|---|
| C | 57.43 | 57.19 |
| H | 5.35 | 5.41 |
| N | 7.44 | 7.28 |

Using a procedure analogous to the aforesaid example, the following compounds are prepared:
3-THIOPHENE CARBOXYLIC ACID, 2-[(2-ACETYL AMINO-3-METHOXY BENZOYL)AMINO}-5-METHYL-n.PROPYL ESTER
3-THIOPHENE CARBOXYLIC ACID, 2-[(2-ACETYL AMINO-3-METHOXY BENZOYL)AMINO]-5-METHYL-

tert.BUTYL ESTER
3-THIOPHENE CARBOXYLIC ACID, 2-[(2-ACETYL AMINO-3-METHOXY BENZOYL)AMINO]-5-ETHYL-ETHYL ESTER
3-THIOPHENE CARBOXYLIC ACID, 2-[(2-ACETYL AMINO-3-METHOXY BENZOYL)AMINO]-5-n.BUTYL-METHYL ESTER
3-THIOPHENE CARBOXYLIC AC ID, 2-[(2-ACETYL AMINO-3-METHYL BENZOYL)AMINO]-5-METHYL-n.PROPYL ESTER
3-THIOPHENE CARBOXYLIC ACID, 2-[(2-ACETYL AMINO-3-METHYL BENZOYL)AMINO]-5-METHYL-tert.BUTYL ESTER
3-THIOPHENE CARBOXYLIC ACID, 2-[(2-ACETYL AMINO-3-METHYL BENZOYL)AMINO]-5-n.BUTYL-METHYL ESTER
3-THIOPHENE CARBOXYLIC ACID, 2-[(2-ACETYL AMINO-5-METHYL BENZOYL)AMINO]-5-ETHYL-ETHYL ESTER
3-THIOPHENE CARBOXYLIC ACID, 2-[(2-ACETYL AMINO-3-METHYL BENZOYL)AMINO]-5-n.PROPYL-ISOPROPYL ESTER
3-THIOPHENE CARBOXYLIC ACID, 2-[(2-ACETYL AMINO-3-METHYL BENZOYL)AMINO]-5-ETHYL-n.OCYTL ESTER

EXAMPLE 10

3-THIOPHENE CARBOXYLIC ACID, 2-[(2-AMINO-3-METHOXY BENZOYL)AMINO]-5-METHYL (No. 16 of Table 1)

0.329 g of sodium hydroxide (0.0082 moles) dissolved in 12 ml of 95% ethanol are added to 0.500 g of 3-thiophene carboxylic acid, 2-[(2-acetyl amino-3-methoxy benzoyl)amino]-5-methyl, ethyl ester (0.00133 moles) (prepared in accordance with Example 9) dissolved in 20 ml of 95% ethanol.

The mixture is heated under reflux for 2 days. The solvent is evaporated and the residue dissolved in 45 ml of water and treated with 45 ml of chloroform.

The aqueous phase is separated, to which 37% hydrochloric acid is then added to pH 1. The precipitate is filtered off through a Buchner funnel. After drying , 0.267 g of 3-thiophene carboxylic acid, 2-[-(2-amino-3-methoxy benzoyl)amino]-5-methyl are obtained (Yield 65.6%, M.P. 241-243 ° C).

| Analysis for $C_{14}H_{14}N_2O_4S$ | % calculated | % found |
|---|---|---|
| C | 54.88 | 55.08 |
| H | 4.60 | 4.68 |
| N | 9.14 | 9.21 |

Using a procedure analogous to the aforesaid example, the following compounds are prepared:
3-THIOPHENE CARBOXYLIC ACID, 2-[(2-AMINO-3-METHOXY BENZOYL) AMINO]-5-METHYL
3-THIOPHENE CARBOXYLIC ACID, 2-[(2-AMINO-3-METHOXY BENZOYL) AMINO]-5-ETHYL
3-THIOPHENE CARBOXYLIC ACID, 2-[(2-AMINO-3-METHOXY BENZOYL) AMINO]-5-n.BUTYL
3-THIOPHENE CARBOXYLIC ACID, 2-[(2-AMINO-3-METHYL BENZOYL) AMINO]-5-METHYL
3-THIOPHENE CARBOXYLIC ACID, 2-[(2-AMINO-3-METHYL BENZOYL) AMINO]-5-n.BUTYL
3-THIOPHENE CARBOXYLIC ACID, 2-[(2-AMINO-5-METHYL BENZOYL) AMINO)-5-ETHYL
3-THIOPHENE CARBOXYLIC ACID, 2-[(2-AMINO-5-METHYL BENZOYL) AMINO]-5-n.PROPYL.

EXAMPLE 11

3-THIOPHENE CARBOXYLIC ACID, 2-[(2-AMINO-3-METHOXY BENZOYL] AMINO]-5-METHYL, SODIUM SALT (No. 17 of Table 1)

40 ml of an 8% sodium hydroxide solution (0.08 are added to a suspension of 4.5 g of 3-thiophene carboxylic acid, 2-[(2-amino-3-methoxy benzoyl)amino]-5-methyl (0.0147 moles) (prepared in accordance with Example 10) in 80 ml of water.

The mixture is heated to 80 ° C for 10 minutes with complete final dissolving.

The solution is cooled, a precipitate forming at ambient temperature. The mixture is diluted with 150 ml

of water and filtered through a Buchner funnel.

After drying, 3.063 g of 3-thiophene carboxylic acid, 2-[(2-amino-3-methoxy benzoyl)amino]-5-methyl, sodium salt are obtained. (Yield 63.5%, M.P. 240°C dec.).

| Analysis for $C_{14}H_{13}N_2O_4SNa$ | % calculated | % found |
|---|---|---|
| C | 51.21 | 51.190 |
| H | 3.99 | 3.96 |
| N | 8.53 | 8.49 |

Using a procedure analogous to the aforesaid example, the following compounds are prepared:

3-THIOPHENE CARBOXYLIC ACID, 2-[(2-AMINO-3-METHOXY BENZOYL) AMINO]-5-METHYL, SODIUM SALT

3-THIOPHENE CARBOXYLIC ACID, 2-[(2-AMINO-3-METHOXY BENZOYL) AMINO)-5-ETHYL, SODIUM SALT

3-THIOPHENE CARBOXYLIC ACID, 2-[(2-AMINO-3-METHOXY BENZOYL) AMINO]-5-n.BUTYL, SODIUM SALT

3-THIOPHENE CARBOXYLIC ACID, 2-[(2-AMINO-3-METHYL BENZOYL) AMINO]-5-METHYL, SODIUM SALT

3-THIOPHENE CARBOXYLIC ACID, 2-[(2-AMINO-3-METHYL BENZOYL) AMINO]-5-n.BUTYL, SODIUM SALT

3-THIOPHENE CARBOXYLIC ACID, 2-[(2-AMINO-5-METHYL BENZOYL) AMINO]-5-ETHYL, SODIUM SALT

3-THIOPHENE CARBOXYLIC ACID, 2-[(2-AMINO-5-METHYL BENZOYL) AMINO]-5-n.PROPYL, SODIUM SALT.

EXAMPLE 12

3-THIOPHENE CARBOXYLIC ACID, 2-[[2-AMINO(OXOACETIC ACID ETHYL ESTER)-3-METHOXY BENZOYL] AMINO]-5-METHYL, ETHYL ESTER (No. 18 of Table 1)

3 g of 3-thiophene carboxylic acid, 2-[(2-amino-3-methoxy benzoyl)amino]-5-methyl, ethyl ester (prepared in accordance with Example 2) (0.00898 moles) dissolved in 65 ml of anhydrous chloroform and 2.02 g of triethylamine (0.02 moles) are introduced into a dry apparatus and maintained in a stream of nitrogen.

The mixture temperature is reduced to -15°C and 2.73 g of ethyl oxalylchloride (0.02 moles) are dripped in.

On termination of the addition, the mixture temperature is allowed to rise to ambient, and water is added. The organic phase is separated, dried and evaporated to dryness. A solid is obtained which is chromatographed in a silica gel column using ethyl ether/ethyl acetate/N-hexane 8:2:1 as eluent.

The fractions containing the pure product are pooled and evaporated to dryness.

1.87 g of 3-thiophene carboxylic acid, 2-[[2-amino(oxoacetic acid ethyl ester)-3-methoxy benzoyl] amino]-5-methyl, ethyl ester are obtained. (Yield 48%, M.P. 140-142°C).

| Analysis for $C_{20}H_{22}N_2O_7S$ | % calculated | % found |
|---|---|---|
| C | 55.28 | 55.49 |
| H | 5.10 | 5.22 |
| N | 6.45 | 6.36 |

Using a procedure analogous to the aforesaid example, the following compounds are prepared:

3-THIOPHENE CARBOXYLIC ACID, 2-[[2-AMINO(OXOACETIC ACID ETHYL ESTER)-3-METHOXY BENZOYL] AMINO]-5-METHYL, n.PROPYL ESTER

3-THIOPHENE CARBOXYLIC ACID, 2-[[2-AMINO(OXOACETIC ACID ETHYL ESTER)-3-METHOXY BENZOYL] AMINO]-5-METHYL, tert.BUTYL ESTER

3-THIOPHENE CARBOXYLIC ACID, 2-[[2-AMINO(OXOACETIC ACID ETHYL ESTER)-3-METHOXY

BENZOYL] AMINO]-5-ETHYL, ETHYL ESTER
3-THIOPHENE CARBOXYLIC ACID, 2-[[2-AMINO(OXOACETIC ACID ETHYL ESTER)-3-METHOXY BENZOYL] AMINO]-5-n.BUTYL, ETHYL ESTER
3-THIOPHENE CARBOXYLIC ACID, 2-[[2-AMINO(OXOACETIC ACID ETHYL ESTER)-3-METHYL BENZOYL] AMINO]-5-METHYL, n.PROPYL ESTER
3-THIOPHENE CARBOXYLIC ACID, 2-[[2-AMINO(OXOACETIC ACID ETHYL ESTER)-3-METHYL BENZOYL] AMINO]-5-METHYL, tert.BUTYL ESTER
3-THIOPHENE CARBOXYLIC ACID, 2-[[2-AMINO(OXOACETIC ACID ETHYL ESTER)-3-METHYL BENZOYL] AMINO]-5-n.BUTYL, ETHYL ESTER
3-THIOPHENE CARBOXYLIC ACID, 2-[[2-AMINO(OXOACETIC ACID ETHYL ESTER)-5-METHYL BENZOYL] AMINO]-5-ETHYL, ETHYL ESTER
3-THIOPHENE CARBOXYLIC ACID, 2-[[2-AMINO(OXOACETIC ACID ETHYL ESTER)-5-METHYL BENZOYL] AMINO]-5-n.PROPYL, ISOPROPYL ESTER
3-THIOPHENE CARBOXYLIC ACID, 2-[[2-AMINO(OXOACETIC ACID ETHYL ESTER)-5-METHYL BENZOYL] AMINO]-5-ETHYL, n.OCTYL ESTER.

EXAMPLE 13

3-THIOPHENE CARBOXYLIC ACID, 2-[(2-ACETYL AMINO-5-CHLORO BENZOYL)AMINO]-5-METHYL, ETHYL ESTER (No. 19 of Table 1)

A suspension composed of 5 g of 3-thiophene carboxylic acid, 2-[(2-amino-5-chloro benzoyl)amino]-5-methyl, ethyl ester (prepared in accordance with Example 3) (0.0147 moles), 24 ml of acetic acid and 4.56 g of acetic anhydride (0.0447 moles) is heated to 80° C for half hour.

The hot reaction mixture is dripped into 300 ml of water-ice. The solid which precipitates is filtered off through a Buchner funnel and crystallized from 81 ml of ethyl acetate.

After drying, 3.265 g of 3-thiophene carboxylic acid, 2-[(2-acetyl amino-5-chloro benzoyl)amino]-5-methyl, ethyl ester are obtained (Yield 58%, M.P. 162-163° C).

| Analysis for $C_{17}H_{17}N_2O_4ClS$ | % calculated | % found |
|---|---|---|
| C | 53.61 | 53.51 |
| H | 4.50 | 4.48 |
| N | 7.38 | 7.30 |

Using a procedure analogous to the aforesaid example, the following compounds are prepared:
3-THIOPHENE CARBOXYLIC ACID, 2-[(2-ACETYL,AMINO-5-CHLORO BENZOYL)AMINO]-5-METHYL-n.PROPYL ESTER
3-THIOPHENE CARBOXYLIC ACID, 2-[(2-ACETYL AMINO-5-CHLORO BENZOYL)AMINO]-5-n.BUTYL-METHYL ESTER
3-THIOPHENE CARBOXYLIC ACID, 2-[(2-ACETYL AMINO-5-CHLORO BENZOYL)AMINO]-5-ETHYL-ETHYL ESTER
3-THIOPHENE CARBOXYLIC ACID, 2-[(2-ACETYL AMINO-4-CHLORO BENZOYL)AMINO]-5-n.PROPYL-ISOPROPYL ESTER
3-THIOPHENE CARBOXYLIC ACID, 2-[(2-ACETYL AMINO-4-CHLORO BENZOYL)AMINO]-5-n.BUTYL-PENTYL ESTER
3-THIOPHENE CARBOXYLIC ACID, 2-[(2-ACETYL AMINO-4-CHLORO BENZOYL)AMINO]-5-METHYL-tert.BUTYL ESTER
3-THIOPHENE CARBOXYLIC ACID, 2-[(2-ACETYL AMINO-3-CHLORO BENZOYL)AMINO]-5-ETHYL-n.OCTYL ESTER
3-THIOPHENE CARBOXYLIC ACID, 2-[(2-ACETYL AMINO-3-CHLORO BENZOYL)AMINO]-5-n.BUTYL-ETHYL ESTER
3-THIOPHENE CARBOXYLIC ACID, 2-[(2-ACETYL AMINO-3-CHLORO BENZOYL)AMINO]-5-METHYL-tert.BUTYL ESTER.

EXAMPLE 14

4-THIAZOLE ACETIC ACID, 2-[(2-ACETYL AMINO-3-METHOXY BENZOYL)AMINO], ETHYL ESTER (No. 30 of Table 1)

A suspension composed of 5.03 g of 4-thiazole acetic acid, 2-[(2-amino-3-methoxy benzoyl)amino], ethyl ester (0.015 moles) (prepared in accordance with Example 7), 60.36 ml of acetic acid and 4.59 g of acetic anhydride (0.045 moles) is heated to 80° C for one hour. The hot reaction mixture is dripped into 600 ml of water and ice. The solid which precipitates is filtered off through a Buchner funnel and crystallized from 102 ml of ethanol. After drying the solid, 3.5 g of 4-thiazole acetic acid, 2-[(2-acetyl amino-3-methoxy benzoyl)amino], ethyl ester are obtained. (Yield 61.8%, M.P. 145-146° C).

| Analysis for $C_{17}H_{19}N_3O_5S$ | % calculated | % found |
|---|---|---|
| C | 54.1 | 53.93 |
| H | 5.07 | 5.03 |
| N | 11.13 | 11.31 |

Using a procedure analogous to the aforesaid example, the following compounds are prepared:
4-THIAZOLE ACETIC ACID, 2-[(2-ACETYL AMINO-3-CHLORO BENZOYL)AMINO], ETHYL ESTER
4-THIAZOLE ACETIC ACID, 2-[(2-ACETYL AMINO-5-METHYL BENZOYL)AMINO], ETHYL ESTER
4-THIAZOLE CARBOXYLIC ACID, 2-[(2-ACETYL AMINO-4-CHLORO BENZOYL)AMINO]-4-METHYL, ETHYL ESTER
5-THIAZOLE CARBOXYLIC ACID, 2-[(2-ACETYL AMINO-3-METHYL BENZOYL)AMINO]-4-METHYL, ETHYL ESTER
5-THIAZOLE CARBOXYLIC ACID, 2-[(2-ACETYL AMINO-3-METHOXY BENZOYL)AMINO]-4-METHYL, ETHYL ESTER
4-THIAZOLE GLYOXYLIC ACID, 2-[(2-ACETYL AMINO-5-CHLORO BENZOYL)AMINO], ETHYL ESTER
4-THIAZOLE GLYOXYLIC ACID, 2-[(2-ACETYL AMINO-3-METHOXY BENZOYL)AMINO], ETHYL ESTER
4-THIAZOLE GLYOXYLIC ACID, 2-[(2-ACETYL AMINO-5-METHYL BENZOYL)AMINO], ETHYL ESTER.

EXAMPLE 15

8-METHOXY-3-[(3-ETHOXY CARBONYL-5-METHYL)-THIENYL]-2,4 QUINAZOLONE (No. 32 of Table 1)

1,580 g of 3-thiophene carboxylic acid, 2-[(2-amino-3-methoxy benzoyl)amino]-5-methyl, ethyl ester (prepared in accordance with Example 2) (0.0047 moles) dissolved in 45 ml of anhydrous chloroform are introduced into a dry apparatus and maintained in a stream of nitrogen.

9 ml of 20% phosgene in toluene (0.018 moles) are dripped into this solution at ambient temperature.

The solution is heated under reflux for 2 hours. It is cooled, and the solvent evaporated.

A solid is obtained which is crystallised from 50 ml of ethanol. The crystallised product is filtered off through a Buchner funnel and dried.

1.282 g of 8-methoxy-3-[(3-ethoxycarbonyl-5-methyl)-thienyl]-2,4 quinazolone are obtained. (Yield 75.7%, M.P. 214-215° C).

| Analysis for $C_{17}H_{16}N_2O_5S$ | % calculated | % found |
|---|---|---|
| C | 56.65 | 56.57 |
| H | 4.47 | 4.46 |
| N | 7.77 | 7.67 |

Using a procedure analogous to the aforesaid example, the following compounds are prepared:
8-CHLORO-3-[(3-ETHOXY CARBONYL-5-METHYL)-THIENYL]-2,4 QUINAZOLONE
8-METHYL-3-[(3-ETHOXY CARBONYL-5-METHYL)-THIENYL]-2,4 QUINAZOLONE
8-CHLORO-3-[(3-METHOXY CARBONYL-5-n.BUTYL)-THIENYL]-2,4 QUINAZOLONE
9-CHLORO-3-[(3-ETHOXY CARBONYL-5-METHYL)-THIENYL]-2,4 QUINAZOLONE
9-CHLORO-3-[(3-METHOXY CARBONYL-5-n.BUTYL)-THIENYL]-2,4 QUINAZOLONE

10-CHLORO-3-[(3-ETHOXY CARBONYL-5-METHYL)-THIENYL]-2,4 QUINAZOLONE
10-METHYL-3-[(3-ETHOXY CARBONYL-5-METHYL)-THIENYL]-2,4 QUINAZOLONE
10-METHYL-3-[(3-METHOXY CARBONYL-5-n.BUTYL)-THIENYL]-2,4 QUINAZOLONE

EXAMPLE 16

8-METHOXY-3-[(5-ETHOXY CARBONYL-4-METHYL)-THIAZENYL]-2,4 QUINAZOLONE (No. 34 of Table 1)

7 g of 5-thiazole carboxylic acid, 2-[(2-amino-3-methoxy benzoyl)amino]-4-methyl, ethyl ester (0.0209 moles) (prepared in accordance with Example 6) dissolved in 130 ml of anhydrous chloroform are introduced into a dry apparatus and maintained in a stream of nitrogen. 39.75 ml of 20% phosgene in toluene (0.08 moles) are then dripped in at ambient temperature and the mixture left under agitation for one hour. On termination, the solvent is evaporated and the solid obtained is crystallised from 190 ml of absolute ethanol. The precipitate is filtered off through a Buchner funnel and dried.

4.725 g of 8-methoxy-3-[(5-ethoxycarbonyl-4-methyl)-thiazenyl]-2,4 quinazolone are obtained. (Yield 62.9%, M.P. 204-206 ° C).

| Analysis for $C_{16}H_{15}N_3O_5S$ | % calculated | % found |
|---|---|---|
| C | 53.17 | 53.38 |
| H | 4.18 | 4.22 |
| N | 11.62 | 11.81 |

Using a procedure analogous to the aforesaid example, the following compounds are prepared:
8-CHLORO-3-[(5-ETHOXY CARBONYL-4-METHYL)-THIAZENYL]-2,4 QUINAZOLONE
8-METHYL-3-[(5-ETHOXY CARBONYL-4-METHYL)-THIAZENYL]-2,4 QUINAZOLONE
9-CHLORO-3-[(4-ACETIC ACID ETHYL ESTER)-THIAZENYL]-2,4 QUINAZOLONE
10-METHYL-3-[(4-ACETIC ACID ETHYL ESTER)-THIAZENYL]-2,4 QUINAZOLONE
9-CHLORO-3-[(4-GLYOXYLIC ACID ETHYL ESTER)-THIAZENYL]-2,4 QUINAZOLONE
10-CHLORO-3-[(4-GLYOXYLIC ACID ETHYL ESTER)-THIAZENYL]-2,4 QUINAZOLONE

EXAMPLE 17

4-THIAZOLE-α-HYDROXY ACETIC ACID, 2-[(2-AMINO-3-METHOXY BENZOYL)AMINO]-ETHYL ESTER (No. 35 of Table 1)

15 g of 4-thiazole glyoxylic acid, 2-[(2-nitro-3-methoxy benzoyl)amino], ethyl ester (prepared in accordance with Example 8) (0.0395 moles) dissolved in 500 ml of tetrahydrofuran are introduced into a Parr apparatus for hydrogenation under pressure, 5 g of 10% palladium on carbon are added to the mixture and hydrogenation is carried out while maintaining the hydrogen pressure around 51 psi = 3.47 atm. On termination of the reaction, the catalyst is filtered off through celite and the solvent evaporated.

The solid residue is chromatographed through a silica gel column using chloroform/methanol 9.5:0.5 as eluent.

The fractions containing the pure product are pooled and evaporated to dryness.

7.8 g of 4-thiazole-α-hydroxyacetic acid, 2-[(2-amino-3-methoxy benzoyl)amino]-ethyl ester are obtained. (Yield 56%, M.P. 180-182 ° C).

| Analysis for $C_{15}H_{17}N_3O_5S$ | % calculated | % found |
|---|---|---|
| C | 51.27 | 50.98 |
| H | 4.87 | 4.76 |
| N | 11.92 | 11.79 |

Using a procedure analogous to the aforesaid example, the following compounds are prepared:
4-THIAZOLE-α-HYDROXY ACETIC ACID-2-[(2-AMINO-3-CHLORO BENZOYL) AMINO]-ETHYL ESTER

4-THIAZOLE-α-HYDROXY ACETIC ACID-2-[(2-AMINO-4-CHLORO BENZOYL) AMINO]-ETHYL ESTER
4-THIAZOLE-α-HYDROXY ACETIC ACID-2-[(2-AMINO-5-CHLORO BENZOYL) AMINO]-ETHYL ESTER
4-THIAZOLE-α-HYDROXY ACETIC ACID-2-[(2-AMINO-3-METHYL BENZOYL) AMINO]-ETHYL ESTER
4-THIAZOLE-α-HYDROXY ACETIC ACID-2-[(2-AMINO-5-METHYL BENZOYL) AMINO]-ETHYL ESTER.

EXAMPLE 18

8-METHOXY-3-[[5-(α-HYDROXY ETHYL ACETATE)]-THIAZENYL]-2,4 QUINAZOLONE (No. 36 of Table 1)

1.052 g of 4-thiazole-α-hydroxy acetic acid, 2-[(2-amino-3-methoxy benzoyl)amino], ethyl ester (0.003 moles) (prepared in accordance with Example 17) dissolved in 35 ml of anhydrous tetrahydrofuran are introduced into a dry apparatus and maintained in a stream of nitrogen. 2.95 ml of 20% phosgene in toluene (0.006 moles) are then dripped in and the mixture left under agitation for one hour. On termination, the solvent is evaporated and the solid obtained is chromatographed in a silica gel column using chloroform/methanol 9.5:0.5 as eluent.

The fractions containing the pure product are pooled and evaporated to dryness.

0.680 g of 8-methoxy-3-[[5-(α-hydroxy ethylacetate)]-thiazenyl]-2,4 quinazolone are obtained. (Yield 60%, M.P. 177-178° C).

| Analysis for $C_{16}H_{15}N_3O_6S$ | % calculated | % found |
|---|---|---|
| C | 50.92 | 50.74 |
| H | 4.00 | 3.97 |
| N | 11.13 | 10.98 |

Using a procedure analogous to the aforesaid example, the following compounds are prepared:
8-CHLORO-3-[[5-(α-HYDROXY ETHYL ACETATE)]-THIAZENYL]-2,4 QUINAZOLONE
9-CHLORO-3-[[5-(α-HYDROXY ETHYL ACETATE)]-THIAZENYL]-2,4 QUINAZOLONE
10-CHLORO-3-[[5-(α-HYDROXY ETHYL ACETATE)]-THIAZENYL]-2,4 QUINAZOLONE
8-METHYL-3-[[5-(α-HYDROXY ETHYL ACETATE)]-THIAZENYL]-2,4 QUINAZOLONE
10-METHYL-3-[[5-(α-HYDROXY ETHYL ACETATE)]-THIAZENYL]-2,4 QUINAZOLONE

EXAMPLE 19

3-THIOPHENE CARBOXYLIC ACID-2-[(2-AMINO-3-HYDROXY BENZOYL)AMINO]-5-METHYL-ETHYL ESTER (No. 12 of Table 1)

3-hydroxy-2-nitro-ethyl benzoate:

100 ml of absolute ethyl alcohol are introduced into a reaction flask, after which 5 ml of concentrated sulphuric acid are added.

10 g of 2-hydroxy-2-nitro-benzoic acid (0.054 moles) are added to this mixture, and the mixture heated under reflux for 5 hours.

It is then left at ambient temperature under agitation overnight, and the solid is then filtered off through a Buchner funnel and washed with water until neutral.

After drying, 9.5 g of 3-hydroxy-2-nitro-ethyl benzoate are obtained. (Yield 83. 3%, M.P. 160-162° C).

| Analysis for $C_9H_9NO_5$ | % calculated | % found |
|---|---|---|
| C | 51.18 | 50.98 |
| H | 4.29 | 4.41 |
| N | 6.63 | 6.38 |

3-benzyloxy-2-nitro-ethyl benzoate:

32 ml of anhydrous ethanol are introduced into a two-neck 500 ml flask fitted with a reflux condenser and maintained in a stream of nitrogen, 1.1 g of sodium metal (0.047 moles) then being added under agitation.

After complete formation of the sodium ethylate, 10 g of 3-hydroxy-2-nitro-ethyl benzoate (0.047 moles) dissolved in 200 ml of anhydrous ethanol are added. After some minutes, 6.7 g of benzyl chloride (0.053 moles) are added and the mixture, heated under reflux for 4 hours.

It is filtered through celite and the filtrate evaporated. The solid residue is crystallized from ethanol.

10 g of 3-benzyloxy-2-nitro-ethyl benzoate are obtained (Yield 70.6%, M.P. 172-173 ° C).

| Analysis for $C_{16}H_{15}NO_5$ | % calculated | % found |
|---|---|---|
| C | 63.78 | 63.65 |
| H | 5.01 | 4.99 |
| N | 4.65 | 4.50 |

3-benzyloxy-2-nitro-benzoic acid:

20 g of 3-benzyloxy-2-nitro-ethyl benzoate, (0.066 moles) are dissolved in 300 ml of ethanol and introduced into the reaction vessel. 2.64 g of sodium hydroxide (0.066 moles) dissolved in 50 ml of ethanol are then added, and the mixture heated under reflux for 3 hours.

The ethanol is evaporated, and the solid obtained is dissolved in water and acidified to pH 2. The precipitate is filtered off through a Buchner funnel and dried. 12 g of 3-benzyloxy-2-nitro-benzoic acid are obtained. (Yield 66%, M.P. 193-195 ° C).

| Analysis for $C_{14}H_{11}NO_5$ | % calculated | % found |
|---|---|---|
| C | 61.54 | 61.48 |
| H | 4.06 | 4.15 |
| N | 5.12 | 5.23 |

3-benzyloxy-2-nitro-benzoyl chloride:

100 g of 3-benzyloxy-2-nitro-benzoic acid (0.366 moles) are mixed in a dry apparatus under a stream of nitrogen with 435.5 g of freshly distilled thionyl chloride (3.66 moles). The mixture is heated under reflux for 5 hours. It is cooled, and the excess thionyl chloride distilled off with a water pump. The solid residue is taken up in anhydrous n-hexane and then filtered off through a Buchner funnel and dried to obtain 96 g of 3-benzyloxy-2-nitro-benzoyl chloride. (Yield 90%, $Cl^-$ purity 98.8%).

| Analysis for $C_{14}H_{10}ClNO_4$ | % calculated | % found |
|---|---|---|
| C | 57.64 | 57.72 |
| H | 3.45 | 3.61 |
| N | 4.80 | 4.93 |

3-thiophene carboxylic acid-2-[(2-nitro-3-benzyloxy benzoyl) amino]-5-methyl ethyl ester (No. 6 of Table 1):

METHOD A

20.7,g of 3-thiophene carboxylic acid-2-amino-5-methyl, ethyl ester (0.112 moles) dissolved in 250 ml of anhydrous methylene chloride and 29.2 g of triethylamine (0.289 moles) are introduced into a dry apparatus. 39 g of 3-benzyloxy-2-nitro-benzoyl chloride (0.134 moles) dissolved in 400 ml of anhydrous

methylene chloride are dripped into this solution. The mixture is heated under reflux for 7 hours, after which the solvent is evaporated and the solid obtained is stirred in 700 ml of ethanol for 2 hours.

The solid residue is filtered off through a Buchner funnel and dried. 22.4 g of 3-thiophene carboxylic acid-2-[(2-nitro-3-benzyloxy benzoyl)amino]-5-methyl ethyl ester are obtained. (Yield 65. 7%, M.P. 208-210 ° C, U.V. purity 100%).

| Analysis for $C_{22}H_{20}N_2O_6S$ | % calculated | % found |
|---|---|---|
| C | 59.98 | 59.80 |
| H | 4.57 | 4.71 |
| N | 6.36 | 6.26 |

3-thiophene carboxylic acid-2-[(2-amino-3-hydroxy-benzoyl) amino]-5-methyl ethyl ester:

51 g of 3-thiophene carboxylic acid-2-[(2-nitro-3-benzyloxy benzoyl)amino]-5-methyl ethyl ester (0.116 moles) dissolved in 750 ml of tetrahydrofuran and to which 13.8 g of 10% palladium on carbon has been added are introduced into a Parr apparatus for hydrogenation under pressure (51 psi = 3.47 atm).

On completion of hydrogen consumption, the catalyst is removed by filtration and the mixture is evaporated to dryness. The solid obtained is crystallized from 950 ml of ethyl acetate. The crystallised product is filtered off through a Buchner funnel and dried.

34.382 g of 3-thiophene carboxylic acid-2-[(2-amino-3-hydroxy benzoyl)amino]-5-methyl ethyl ester are obtained. (Yield 92.6%, M.P. 220-221 ° C, U.V. purity 99.8%).

| Analysis for $C_{15}H_{16}N_2O_4S$ | % calculated | % found |
|---|---|---|
| C | 56.23 | 56.64 |
| H | 5.03 | 4.88 |
| N | 8.74 | 8.72 |

TABLE 1

| COMPOUND No. | $R_1$ | $R_2$ | $R_3$ | $R_4$ | $R_5$ | $R_6$ | Y | C | H | N |
|---|---|---|---|---|---|---|---|---|---|---|
| 1 | 3-$OCH_3$ | $C_2H_5$ | H | $CH_3$ | H | $NO_2$ | C-R | 52.73<br>52.68 | 4.42<br>4.51 | 7.68<br>7.73 |
| 2 | 3-Cl | $C_2H_5$ | H | $CH_3$ | H | $NO_2$ | C-R | 48.84<br>48.99 | 3.55<br>3.21 | 7.59<br>7.71 |
| 3 | 3-$CH_3$ | $C_2H_5$ | H | $CH_3$ | H | $NO_2$ | C-R | 55.16<br>54.83 | 4.63<br>4.51 | 8.04<br>8.12 |
| 4 | 5-Cl | $C_2H_5$ | H | $CH_3$ | H | $NO_2$ | C-R | 48.84<br>49.01 | 3.55<br>3.58 | 7.59<br>7.62 |
| 5 | 5-$CH_3$ | $C_2H_5$ | H | $CH_3$ | H | $NO_2$ | C-R | 55.16<br>55.28 | 4.63<br>4.48 | 8.04<br>8.24 |
| 6 | 3-OBz | $C_2H_5$ | H | $CH_3$ | H | $NO_2$ | C-R | 59.85<br>59.80 | 4.80<br>4.71 | 6.34<br>6.26 |
| 7 | 3-$OCH_3$ | $C_2H_5$ | H | $CH_3$ | H | $NH_2$ | C-R | 57.46<br>57.29 | 5.42<br>5.43 | 8.37<br>8.38 |
| 8 | 3-Cl | $C_2H_5$ | H | $CH_3$ | H | $NH_2$ | C-R | 53.17<br>52.98 | 4.46<br>4.32 | 8.27<br>8.41 |
| 9 | 3-$CH_3$ | $C_2H_5$ | H | $CH_3$ | H | $NH_2$ | C-R | 60.35<br>59.98 | 5.70<br>5.61 | 8.80<br>8.75 |
| 10 | 5-Cl | $C_2H_5$ | H | $CH_3$ | H | $NH_2$ | C-R | 53.17<br>53.06 | 4.46<br>4.48 | 8.27<br>8.32 |
| 11 | 5-$CH_3$ | $C_2H_5$ | H | $CH_3$ | H | $NH_2$ | C-R | 60.35<br>60.55 | 5.70<br>5.84 | 8.80<br>8.61 |
| 12 | 3-OH | $C_2H_5$ | H | $CH_3$ | H | $NH_2$ | C-R | 56.23<br>56.64 | 5.03<br>4.88 | 8.74<br>8.72 |
| 13 | 5-$CH_3$ | $CH_3$ | H | n $C_4H_9$ | H | $NO_2$ | C-R | 57.43<br>57.39 | 5.35<br>5.34 | 7.44<br>7.50 |
| 14 | 5-$CH_3$ | $CH_3$ | H | n $C_4H_9$ | H | $NH_2$ | C-R | 62.40<br>61.90 | 6.40<br>6.28 | 8.08<br>8.10 |
| 15 | 3-$OCH_3$ | $C_2H_5$ | H | $CH_3$ | H | NH-CO-$CH_3$ | C-R | 57.43<br>57.19 | 5.35<br>5.41 | 7.44<br>7.28 |
| 16 | 3-$OCH_3$ | H | H | $CH_3$ | H | $NH_2$ | C-R | 54.88<br>55.08 | 4.60<br>4.68 | 9.14<br>9.21 |
| 17 | 3-$OCH_3$ | Na | H | $CH_3$ | H | $NH_2$ | C-R | 51.21<br>51.19 | 3.99<br>3.96 | 8.53<br>8.49 |
| 18 | 3-$OCH_3$ | $C_2H_5$ | H | $CH_3$ | H | NH-CO-CO-O$C_2H_5$ | C-R | 55.28<br>55.49 | 5.10<br>5.22 | 6.45<br>6.36 |

TABLE 1 (continued)

| COMPOUND No. | $R_1$ | $R_2$ | $R_3$ | $R_4$ | $R_5$ | $R_6$ | Y | C | H | N |
|---|---|---|---|---|---|---|---|---|---|---|
| 19 | 5-Cl | $C_2H_5$ | H | $CH_3$ | H | NH-CO-$CH_3$ | C-R | 53,61<br>53,51 | 4,50<br>4,48 | 7,38<br>7,30 |
| 20 | 5-$CH_3$ | H | H | $CH_3$ | H | $NH_2$ | C-R | 57,90<br>57,72 | 4.86<br>4.83 | 9.65<br>9,44 |
| 21 | 5-$CH_3$ | $CH_3$ | H | n$C_4H_9$ | H | NH-CO-CO-$OC_{26}$ | C-R | 59,17<br>59,03 | 5,87<br>5,69 | 6,27<br>6.32 |
| 22 | 3-$OCH_3$ | - | $CH_3$ | CO-$OC_2H_5$ | H | $NO_2$ | N | 49,31<br>49,21 | 4,14<br>4.08 | 11.50<br>11.36 |
| 23 | 3-$OCH_3$ | - | $CH_3$ | CO-$OC_2H_5$ | H | $NH_2$ | N | 53.71<br>53,57 | 5.11<br>4.99 | 12.53<br>12.61 |
| 24 | 3-$OCH_3$ | - | $CH_2$-CO-$OC_2H_5$ | H | H | $NO_2$ | N | 49.31<br>49,51 | 4,14<br>4,08 | 11,50<br>11.38 |
| 25 | 3-$OCH_3$ | - | $CH_2$-CO-$OC_2H_5$ | H | H | $NH_2$ | N | 53.71<br>53.42 | 5.11<br>5.21 | 12,53<br>12.40 |
| 26 | 3-$OCH_3$ | - | CO-CO-$OC_2H_5$ | H | H | $NO_2$ | N | 47,49<br>47.26 | 3.45<br>3.35 | 11,07<br>11.01 |
| 27 | 3-$OCH_3$ | - | CO-CO-$OC_2H_5$ | H | H | $NH_2$ | N | 51.56<br>51,49 | 4.32<br>4,33 | 12,02<br>11.98 |
| 28 | 3-$OCH_3$ | - | $CH_3$ | CO-$OC_2H_5$ | H | NH-CO-$CH_3$ | N | 54.10<br>53.86 | 5.07<br>5,21 | 11.13<br>11,24 |
| 29 | 5-Cl | - | $CH_3$ | CO-$OC_2H_5$ | H | NH-CO-CO-$OC_2H_5$ | N | 49.15<br>48.96 | 4,12<br>4.36 | 9,55<br>9,28 |
| 30 | 3-$OCH_3$ | - | $CH_2$-CO-$OC_2H_5$ | H | H | NH-CO-$CH_3$ | N | 54.10<br>53.93 | 5.07<br>5.03 | 11.13<br>11,31 |
| 31 | 3-$OCH_3$ | - | CO-CO-$OC_2H_5$ | H | H | NH-CO-CO-$OC_2H_5$ | N | 50.77<br>50,91 | 4.26<br>4.12 | 9.35<br>9,62 |
| 32 | 3-$OCH_3$ | $C_2H_5$ | H | $CH_3$ | $R_5$, $R_6$ = (-CO-NH) | | C-R | 56.65<br>56,57 | 4.47<br>4,46 | 7,77<br>7.67 |
| 33 | 5-Cl | $CH_3$ | H | n$C_4H_9$ | $R_5$, $R_6$ = (-CO-NH) | | C-R | 55,02<br>55,12 | 4.36<br>4.53 | 7.13<br>7.20 |
| 34 | 3-$OCH_3$ | - | $CH_3$ | $COOC_2H_5$ | $R_5$, $R_6$ = (-CO-NH) | | N | 53,17<br>53,38 | 4.18<br>4,22 | 11.62<br>11.81 |
| 35 | 3-$OCH_3$ | - | CHOH-CO-$OC_2H_5$ | - | H | $NH_2$ | N | 51,27<br>50,98 | 4.87<br>4.76 | 11,92<br>11,79 |
| 36 | 3-$OCH_3$ | - | CHOH-CO-$OC_2H_5$ | H | $R_5$, $R_6$ = (-CO-NH) | | N | 50,92<br>50,74 | 4,00<br>3,91 | 11.13<br>10,98 |

TABLE 1 (continued)

| COMPOUND No. | $R_1$ | $R_2$ | $R_3$ | $R_4$ | $R_5$ | $R_6$ | Y | C | H | N |
|---|---|---|---|---|---|---|---|---|---|---|
| 37 | 5-$CH_3$ | - | $CH_2$-CO-$OC_2H_5$ | H | H | $NH_2$ | N | 56.40<br>56.21 | 5.36<br>5.31 | 13.15<br>13.09 |
| 38 | 5-$CH_3$ | - | $CH_2$-CO-$OC_2H_5$ | H | H | NH-CO-$CH_3$ | N | 56.49<br>56.37 | 5.29<br>5.33 | 11.62<br>11.65 |

PHARMACOLOGICAL TRIALS

The products of general formula (I) listed in Table 1 were subjected to a first pharmacological screening to evaluate their activity after oral administration in the passive cutaneous anaphylaxis test (PCA) in the rat in accordance with Goose J. and Blair A.M.J.N. (Immunology, 16, 749-760, 1969).

Serum, obtained from rats actively sensitized with ovalbumin and suitably diluted, was injected intradermally into the shave back of male Sprague Dawley rats, and after 24 hours these were treated intravenously with a solution of ovalbumin and Evans blue.

Thirty minutes after the antigen challenge, the animals were sacrificed and the diameter and intensity of the blue halo which has formed in the intradermal injection site was measured.

The compounds according to the invention, and also tranilast (N-3′, 4′-dimethoxycinnamoyl) anthranilic acid) used as reference standard, were dissolved-suspended in suitable solvents (carboxymethylcellulose, gum arabic, DMSO etc.) and administered orally thirty minutes before the antigen challenge.

Table 2 lists the $ED_{50}$ values and the relative confidence limits at $p = 0.05$.

TABLE 2

| Results of antiallergic activity evaluation in the PCA test on the rat after oral administration 30 minutes before antigen challenge | | | | |
|---|---|---|---|---|
| Compound No. | Molecular weight | $ED_{50}$ mmoles/kg/os | Confidence limits mmoles/kg/os | Activity |
| Tranilast | 327 | 0.32 | 0.09-1.17 | 1 |
| 1 | 364 | 0.21 | 0.08-0.51 | 1.5 |
| 7 | 334 | $0.71 \times 10^{-3}$ | $0.18\text{-}2.6 \times 10^{-3}$ | 450.7 |
| 16 | 306 | $7.8 \times 10^{-3}$ | $3.0\text{-}19.0 \times 10^{-3}$ | 41.0 |
| 15 | 376 | 0.013 | 0.004-0.048 | 24.6 |
| 18 | 434 | $2.8 \times 10^{-3}$ | $0.81\text{-}10.0 \times 10^{-3}$ | 114.3 |
| 32 | 360 | 0.08 | 0.02-0.029 | 4.0 |
| 25 | 335 | $5.0 \times 10^{-3}$ | $1.4\text{-}18.0 \times 10^{-3}$ | 64.0 |
| 37 | 319 | $1.5 \times 10^{-3}$ | $0.4\text{-}5.4 \times 10^{-3}$ | 213.3 |
| 38 | 361 | $6.6 \times 10^{-3}$ | $2.5\text{-}16.0 \times 10^{-3}$ | 48.4 |
| 36 | 377 | 0.054 | 0.017-0.019 | 5.9 |

Some of the compounds according to the invention were subjected to a further passive cutaneous anaphylaxis test evaluation to determine their efficiency after intraperitoneal administration at a dose of 100 mg/kg, fifteen minutes before the challenge.

Table 3 shows the percentage variations calculated for each treatment group (6 in number) with respect to the control group (6 in number).

TABLE 3

| Results of antiallergic activity evaluation in the PCA test on the rat after intraperitoneal administration 15 minutes before antigen challenge | | |
|---|---|---|
| Compound No. | Molecular weight | Inhibition % |
| 1 | 364 | 59.4 |
| 7 | 334 | 31.9 |
| 32 | 360 | 52.7 |
| 25 | 335 | 43.1 |
| 37 | 319 | 88.4 |
| 36 | 377 | 40.7 |

As can be observed from the results given in Tables 2 and 3, the compounds according to the invention are able to inhibit passive cutaneous anaphylaxis in the rat when administered both orally and intraperitoneally.

In particular, when administered orally the results are superior to tranilast by up to three orders of magnitude.

The acute toxicity ($LD_{50}$) of the products according to the invention is considerably greater than 500 mg/kg both for oral administration and for intraperitoneal administration, in that at this dose (the maximum dose tested) no toxic effects were ever manifested.

## Claims

## Claims for the following Contracting States : BE, CH, DE, FR, GB, IT, LI, LU, NL, SE

**1.** Heterocyclic amido derivatives of substituted benzoic acids and therapeutically acceptable salts thereof, of general formula (I):

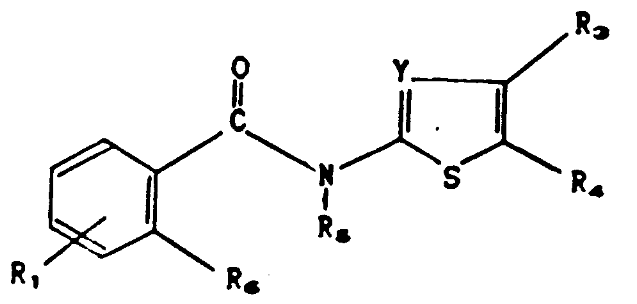

(I)

in which:

- $R_1$ is H; $3\text{-}OCH_3$; 3-Cl; 4-Cl; 5-Cl; $3\text{-}CH_3$; $5\text{-}CH_3$; or 3-OH;
- $R_3$ is H; $-CH_3$; -CO-COOH; $-CO\text{-}CO_2\text{-}C_2H_5$;

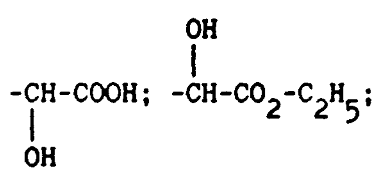

$-CH_2\text{-}COOH$; or $-CH_2\text{-}CO_2\text{-}C_2H_5$;
- $R_4$ is H; a linear alkyl radical of 1-4 carbon atoms; -COOH; or $-CO_2C_2H_5$;
- $R_5$ is H, or $R_5$ and $R_6$ together form (-CO-NH);
- $R_6$ is H; $-NO_2$; $-NH_2$ -NH-COH; $-NH\text{-}COCH_3$; $-NH\text{-}CO\text{-}CO(CH_2)_n\text{-}CH_3$ where n is 0, 1, 2 or 3; or $R_6$ ad $R_5$ together form (NH-CO-);
- Y is N; or C-R in which R is $CO_2R_2$ and $R_2$ is H or an alkaline or alkaline-earth metal equivalent or a linear or branched alkyl radical containing 1-8 carbon atoms;

with the following provisos:

(a) when $R_1 = R_5 = R_6 = R_3 = R_4 = H$ Y is ≠ N
(b) when $R_1 = R_3 = R_4 = H$, $R_5$ and $R_6$ together form -CO-NH-, Y is ≠ N
(c) when $R_1 = R_5 = R_6 = H$, $R_3 = CH_3$ and $R_4 = H$ or $CH_3$, Y is ≠ -C-COOET
(d) when $R_1 = R_5 = R_6 = H$ and $R_3 = R_4 = CH_3$ Y is ≠ -C-COOH
(e) when $R_1 = R_5 = R_6 = H$
$R_3 = H$, and $R_4 = H$,Me,Et,Bu Y ≠ -C-COOET
(f) when $R_1 = R_5 = R_6 = R_3 = H$
and $R_4 = H$,Me,Bu Y ≠ -C-COOMe

**2.** A process for preparing heterocyclic amido derivatives of substituted benzoic acids of general formula (I):

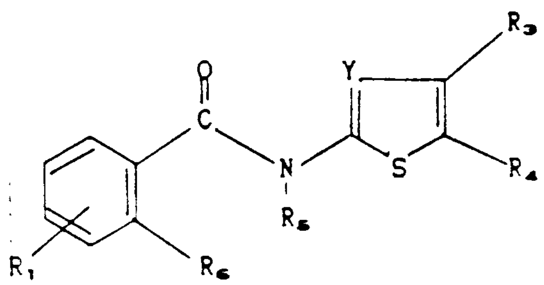

(I)

in which:

$R_1$ is H; 3-$OCH_3$; 3-Cl; 4-Cl; 5-Cl; 3-$CH_3$; 5-$CH_3$; or 3-OH;
$R_3$ is H; -$CH_3$; -CO-COOH; -CO-$CO_2$-$C_2H_5$;

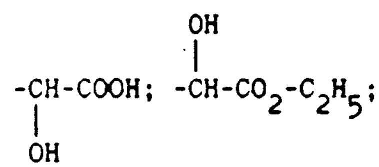

-$CH_2$-COOH; or -$CH_2$-$CO_2$-$C_2H_5$;
$R_4$ is H; a linear alkyl radical of 1-4 carbon atoms; -COOH; or -$CO_2C_2H_5$;
$R_5$ is H, or $R_5$ and $R_6$ together form (-CO-NH);
$R_6$ is H; -$NO_2$; -$NH_2$; -NH-COH; -NH-$COCH_3$; -NH-CO-CO-$(CH_2)_n$-$CH_3$ where n is 0, 1, 2 or 3; or $R_6$ ad $R_5$ together form (NH-CO-);
Y is N; or C-R in which R is $CO_2R_2$ and $R_2$ is H or an alkaline or alkaline-earth metal equivalent or a linear or branched alkyl radical containing 1-8 carbon atoms;

with the following provisos:

(a) when $R_1 = R_5 = R_6 = R_3 = R_4 = H$ Y is ≠ N
(b) when $R_1 = R_3 = R_4 = H$, $R_5$ and $R_6$ together form -CO-NH-, Y is ≠ N
(c) when $R_1 = R_5 = R_6 = H$, $R_3 = CH_3$ and $R_4 = H$ or $CH_3$, Y is ≠ -C-COOET
(d) when $R_1 = R_5 = R_6 = H$ and $R_3 = R_4 = CH_3$ Y is ≠ -C-COOH
(e) when $R_1 = R_5 = R_6 = H$
$R_3 = H$, and $R_4 = H$,Me,Et,Bu Y ≠ -C-COOET
(f) when $R_1 = R_5 = R_6 = R_3 = H$
and $R_4 = H$,Me,Bu Y ≠ -C-COOMe

comprising

a) condensing substituted o-nitrobenzoic or p-nitrobenzoic acid (II)

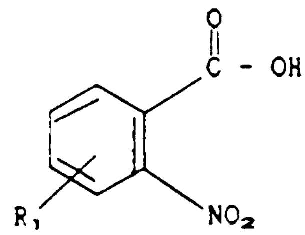

(II)

or the corresponding chloride (V)

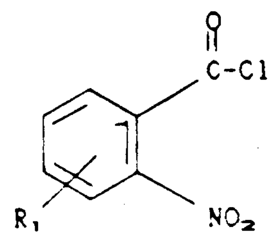

(V)

with a 2-aminothiophene derivative of general formula (III)

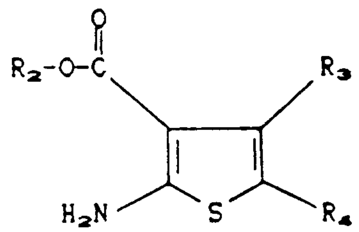

(III)

or a 2-aminothiazole derivative of general formula (IV)

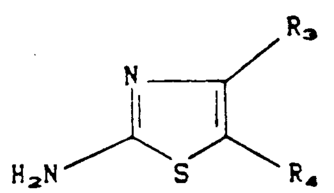

(IV)

b) possibly reducing the nitro group bonded to the aromatic ring to an amino group, and c) possibly functionalising this amino group.

**3.** A process as claimed in claim 2, characterised in that the condensation of (V) with (III) or with (IV) is conducted in aprotic organic solvents in the presence of basic substances at a temperature of between 40 and 160 °C, and preferably between 40 and 70 °C.

**4.** A process as claimed in claim 3, characterised in that said aprotic organic solvents are $CHCl_3$, $CH_2Cl_2$ acetone, benzene, toluene, THF, dioxane and DMF, and are preferably $CH_2Cl_2$ and $CHCl_3$.

**5.** A process as claimed in claim 3, characterised in that said basic substances are $Me_3N$, $Et_3N$, pyridine, $K_2CO_3$ and $Na_2CO_3$, and are preferably triethylamine and potassium carbonate.

**6.** A process as claimed in claim 2, characterised in that said condensation reaction is conducted with a molar ratio of (V) to (III), or of (V) to (IV), of between 1 and 2.5, and preferably between 1 and 1.2.

**7.** A process as claimed in claim 2, characterised in that the condensation of (II) with (III) or with (IV) is conducted in a reaction medium consisting of organic solvents in the presence of condensing agents,

at a temperature of between 0 and 60 ° C.

**8.** A process as claimed in claim 7, characterised in that the organic solvents used are EtOH, THF, benzene and dioxane, and preferably THF.

**9.** A process as claimed in claim 7, characterised in that the condensing agents used are dicyclohexylcarbodiimide, ethoxyacetylene, diethylcyanamide, and triphenyl phosphite + imidazole, and preferably dicyclohexylcarbodiimide.

**10.** A process as claimed in claim 7, characterised in that the molar ratio of compound (II) to compound (III), or of compound (II) to compound (IV) is between 0.5 and 1, and preferably between 0.5 and 0.67.

**11.** A process for preparing heterocyclic amido derivatives of substituted benzoic acids of general formula (I) in which $R_5$ and $R_6$ form NH-CO to give tricyclic compounds, characterised by treating with phosgene a compound of general formula (I) in which $R_5$ is H and $R_6$ is $NH_2$.

**12.** A process as claimed in claim 11, characterised in that said treatment is conducted with phosgene dissolved in toluene.

**13.** A process as claimed in claim 11, characterised in that said treatment is conducted at a temperature of between ambient and the reflux boiling temperature.

**14.** The use of heterocyclic amidoderivatives of substituted benzoic acid of general formula (I)

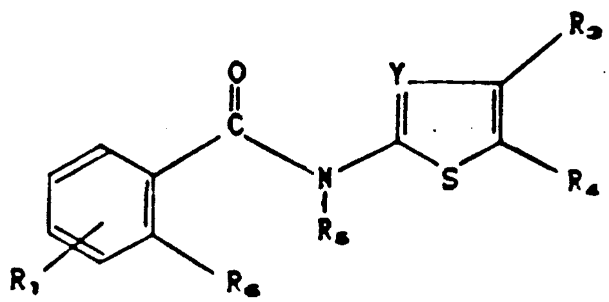

(I)

and of their therapeutically active salts,
in which:

$R_1$ is H; 3-$OCH_3$; 3-Cl; 4-Cl; 5-Cl; 3-$CH_3$; 5-$CH_3$; or 3-OH;

$R_3$ is H; -$CH_3$; -CO-COOH; -CO-$CO_2$-$C_2H_5$;

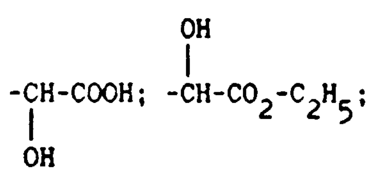

-$CH_2$-COOH; or -$CH_2$-$CO_2$-$C_2H_5$;

$R_4$ is H; a linear alkyl radical of 1-4 carbon atoms; -COOH; or -$CO_2C_2H_5$;

$R_5$ is H, or $R_5$ and $R_6$ together form (-CO-NH);

$R_6$ is H; -$NO_2$; -$NH_2$; -NH-COH; -NH-$COCH_3$; -NH-CO-CO-$(CH_2)_n$-$CH_3$ where n is 0, 1, 2 or 3; or $R_6$ ad $R_5$ together form (NH-CO-);

Y is N; or C-R in which R is $CO_2R_2$ and $R_2$ is H or an alkaline or alkaline-earth metal equivalent or a linear or branched alkyl radical containing 1-8 carbon atoms;

for preparing pharmaceutical composition for the treatment of allergic affections.

**Claims for the following Contracting States : AT, ES, GR**

**1.** A process for preparing heterocyclic amido derivatives of substituted benzoic acids of general formula (I):

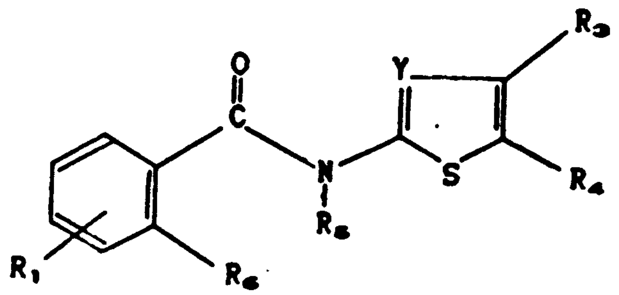

(I)

in which:

$R_1$ is H; 3-$OCH_3$; 3-Cl; 4-Cl; 5-Cl; 3-$CH_3$; 5-$CH_3$; or 3-OH;

$R_3$ is H; -$CH_3$; -CO-COOH; -CO-$CO_2$-$C_2H_5$;

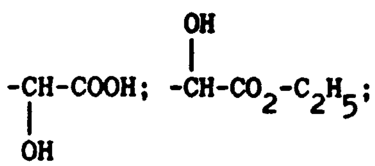

-$CH_2$-COOH; or -$CH_2$-$CO_2$-$C_2H_5$;

$R_4$ is H; a linear alkyl radical of 1-4 carbon atoms; -COOH; or -$CO_2C_2H_5$;

$R_5$ is H, or $R_5$ and $R_6$ together form (-CO-NH);

$R_6$ is H; -$NO_2$; -$NH_2$; -NH-COH; -NH-$COCH_3$; -NH-CO-CO-$(CH_2)_n$-$CH_3$ where n is 0, 1, 2 or 3; or $R_6$ ad $R_5$ together form (NH-CO-);

Y is N; or C-R in which R is $CO_2R_2$ and $R_2$ is H or an alkaline or alkaline-earth metal equivalent or a linear or branched alkyl radical containing 1-8 carbon atoms;

with the following provisos:

with the following provisos:

(a) when $R_1 = R_5 = R_6 = R_3 = R_4 =$ H Y is ≠ N

(b) when $R_1 = R_3 = R_4 =$ H, $R_5$ and $R_6$ together form -CO-NH-, Y is ≠ N

(c) when $R_1 = R_5 = R_6 =$ H, $R_3 = CH_3$ and $R_4 =$ H or $CH_3$, Y is ≠ -C-COOET

(d) when $R_1 = R_5 = R_6 =$ H and $R_3 = R_4 = CH_3$ Y is ≠ -C-COOH

(e) when $R_1 = R_5 = R_6 =$ H

$R_3 =$ H, and $R_4 =$ H,Me,Et,Bu Y ≠ -C-COOET

(f) when $R_1 = R_5 = R_6 = R_3 =$ H

and $R_4 =$ H,Me,Bu Y ≠ -C-COOMe

comprising

a) condensing substituted o-nitrobenzoic or p-nitrobenzoic acid (II)

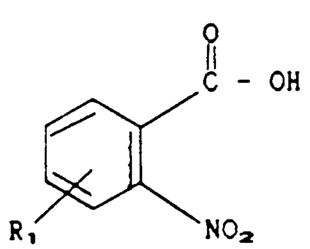

(II)

or the corresponding chloride (V)

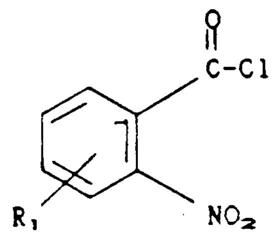

(V)

with a 2-aminothiophene derivative of general formula (III)

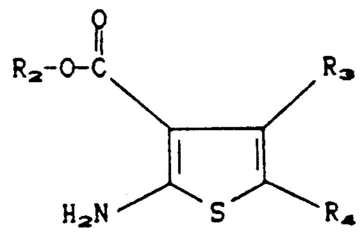

(III)

or a 2-aminothiazole derivative of general formula (IV)

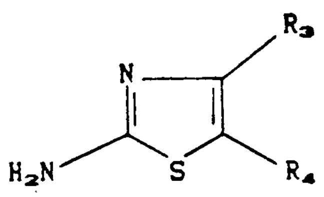

(IV)

b) possibly reducing the nitro group bonded to the aromatic ring to an amino group, and c) possibly functionalising this amino group.

2. A process as claimed in claim 1, characterised in that the condensation of (V) with (III) or with (IV) is conducted in aprotic organic solvents in the presence of basic substances at a temperature of between 40 and 160 ° C, and preferably between 40 and 70 ° C.

3. A process as claimed in claim 2, characterised in that said aprotic organic solvents are $CHCl_3$, $CH_2Cl_2$, acetone, benzene, toluene, THF, dioxane and DMF, and are preferably $CH_2Cl_2$ and $CHCl_3$.

4. A process as claimed in claim 2, characterised in that said basic substances are $Me_3N$, $Et_3N$, pyridine, $K_2CO_3$ and $Na_2CO_3$, and are preferably triethylamine and potassium carbonate.

5. A process as claimed in claim 1, characterised in that said condensation reaction is conducted with a molar ratio of (V) to (III), or of (V) to (IV), of between 1 and 2.5, and preferably between 1 and 1.2.

6. A process as claimed in claim 1, characterised in that the condensation of (II) with (III) or with (IV) is conducted in a reaction medium consisting of organic solvents in the presence of condensing agents, at a temperature of between 0 and 60 ° C.

7. A process as claimed in claim 6, characterised in that the organic solvents used are EtOH, THF, benzene and dioxane, and preferably THF.

8. A process as claimed in claim 6, characterised in that the condensing agents used are dicyclohexylcarbodiimide, ethoxyacetylene, diethylcyanamide, and triphenyl phosphite + imidazole, and preferably dicyclohexylcarbodiimide.

9. A process as claimed in claim 6, characterised in that the molar ratio of compound (II) to compound

(III), or of compound (II) to compound (IV) is between 0.5 and 1, and preferably between 0.5 and 0.67.

**10.** A process for preparing heterocyclic amido derivatives of substituted benzoic acids of general formula (I) in which $R_5$ and $R_6$ form NH-CO to give tricyclic compounds, characterised by treating with phosgene a compound of general formula (I) in which $R_5$ is H and $R_6$ is $NH_2$.

**11.** A process as claimed in claim 10, characterised in that said treatment is conducted with phosgene dissolved in toluene.

**12.** A process as claimed in claim 10, characterised in that said treatment is conducted at a temperature of between ambient and the reflux boiling temperature.

**13.** The use of heterocyclic amidoderivatives of substituted benzoic acid of general formula (I)

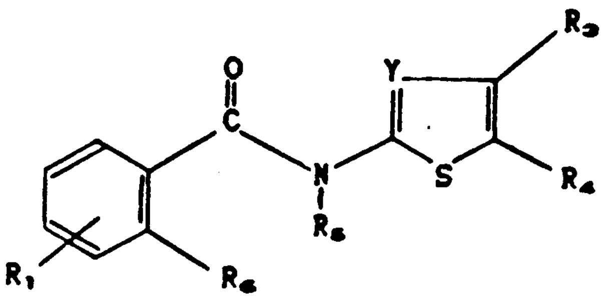

(I)

and of their therapeutically active salts,
in which:

$R_1$ is H; $3\text{-}OCH_3$; 3-Cl; 4-Cl; 5-Cl; $3\text{-}CH_3$; $5\text{-}CH_3$; or 3-OH;

$R_3$ is H; $-CH_3$; -CO-COOH; $-CO\text{-}CO_2\text{-}C_2H_5$;

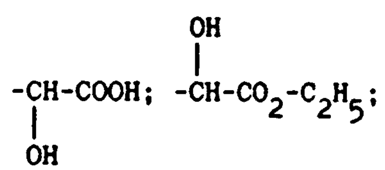

$-CH_2\text{-}COOH$; or $-CH_2\text{-}CO_2\text{-}C_2H_5$;

$R_4$ is H; a linear alkyl radical of 1-4 carbon atoms; -COOH; or $-CO_2C_2H_5$;

$R_5$ is H, or $R_5$ and $R_6$ together form (-CO-NH);

$R_6$ is H; $-NO_2$; $-NH_2$; -NH-COH; $-NH\text{-}COCH_3$; $-NH\text{-}CO\text{-}CO\text{-}(CH_2)_n\text{-}CH_3$ where n is 0, 1, 2 or 3; or $R_6$ ad $R_5$ together form (NH-CO-);

Y is N; or C-R in which R is $CO_2R_2$ and $R_2$ is H or an alkaline or alkaline-earth metal equivalent or a linear or branched alkyl radical containing 1-8 carbon atoms;

for preparing pharmaceutical composition for the treatment of allergic affections.

**Revendications**
**Revendications pour les Etats contractants suivants : BE, CH, DE, FR, GB, IT, LI, LU, NL, SE**

**1.** Dérivés amides hétérocycliques d'acides benzoïques substitués, ainsi que leurs sels thérapeutiquement acceptables, de formule générale (I) :

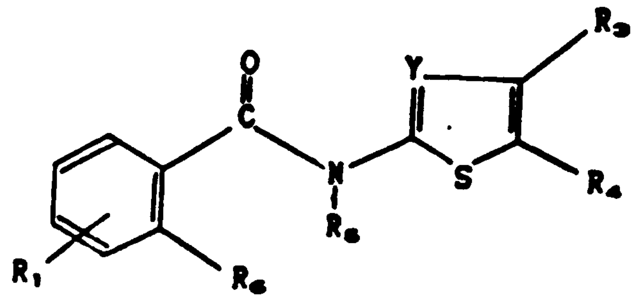

(I)

dans laquelle :
$R_1$ est H; 3-$OCH_3$; 3-Cl; 4-Cl; 5-Cl; 3-$CH_3$; 5-$CH_3$; ou 3-OH;
$R_3$ est H; -$CH_3$; -CO-COOH; -CO-$CO_2$-$C_2H_5$;

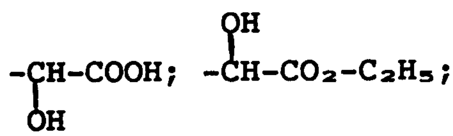

- $CH_2$-COOH; ou -$CH_2$-$CO_2$-$C_2H_5$;
$R_4$ est H; un radical alkyle linéaire ayant de 1 à 4 atomes de carbone; -COOH ou -$CO_2C_2H_5$;
$R_5$ est H, ou $R_5$ et $R_6$ ensemble forment (-CO-NH);
$R_6$ est H; -$NO_2$; -$NH_2$; -NH-COH; -NH-$COCH_3$; -NH-CO-CO-$(CH_2)_n$-$CH_3$ où n vaut 0, 1, 2 ou 3; ou $R_6$ et $R_5$ forment ensemble (NH-CO-);
Y est N; ou C-R dans lequel R est $CO_2R_2$ et $R_2$ est H ou un métal calcalin ou alcalino-terreux équivalent ou un radical alkyle linéaire ou ramifié contenant de 1 à 8 atomes de carbone;
avec les conditions suivantes :

(a) quand $R_1 = R_5 = R_6 = R_3 = R_4 = H$, Y est ≠ N
(b) quand $R_1 = R_3 = R_4 = H$, $R_5$ et $R_6$ forment ensemble -CO-NH-, Y est ≠N
(c) quand $R_1 = R_5 = R_6 = H$, $R_3 = CH_3$ et $R_4$ = H ou $CH_3$, Y est ≠ -C-COOET
(d) quand $R_1 = R_5 = R_6 = H$ et $R_3 = R_4 = CH_3$, Y est ≠ -C-COOH
(e) quand $R_1 = R_5 = R_6 = H$, $R_3 = H$ et $R_4$ = H,Me, Et, Bu; Y ≠ -C-COOET
(f) quand $R_1 = R_5 = R_6 = R_3 = H$ et $R_4$ = H, Me, Bu; Y ≠ -C-COOMe

**2.** Procédé de préparation de dérivés amides hétérocycliques d'acides benzoïques substitués, de formule générale (I) :

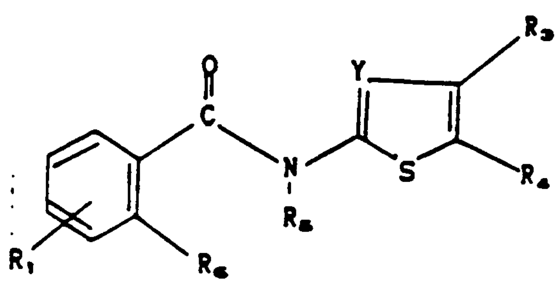

(I)

dans laquelle :
$R_1$ est H; 3-$OCH_3$; 3-Cl; 4-Cl; 5-Cl; 3-$CH_3$; 5-$CH_3$; ou 3-OH;
$R_3$ est H; -$CH_3$; -CO-COOH; -CO-$CO_2$-$C_2H_5$;

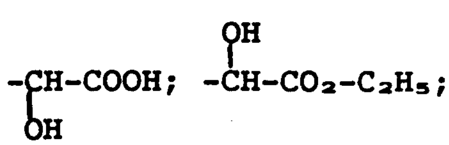

- $CH_2$-COOH; ou -$CH_2$-$CO_2$-$C_2H_5$;

$R_4$ est H; un radical alkyle linéaire ayant de 1 à 4 atomes de carbone; -COOH ou -$CO_2C_2H_5$;

$R_5$ est H, ou $R_5$ et $R_6$ ensemble forment (-CO-NH);

$R_6$ est H; -$NO_2$; -$NH_2$; -NH-COH; -NH-$COCH_3$; -NH-CO-CO-($CH_2$)-$CH_3$ où n vaut 0, 1, 2 ou 3; ou $R_6$ et $R_5$ forment ensemble (NH-CO-);

Y est N; ou C-R dans lequel R est $CO_2R_2$ et $R_2$ est H ou un métal calcalin ou alcalino-terreux équivalent ou un radical alkyle linéaire ou ramifié contenant de 1 à 8 atomes de carbone;

avec les conditions suivantes :

(a) quand $R_1 = R_5 = R_6 = R_3 = R_4 = H$, Y est ≠ M

(b) quand $R_1 = R_3 = R_4 = H$, $R_5$ et $R_6$ forment ensemble -CO-NH-, Y est ≠N

(c) quand $R_1 = R_5 = R_6 = H$, $R_3 = CH_3$ et $R_4$ = H ou $CH_3$, Y est ≠ -C-COOET

(d) quand $R_1 = R_5 = R_6 = H$ et $R_3 = R_4 = CH_3$, Y est = -C-COOH

(e) quand $R_1 = R_5 = R_6 = H$, $R_3$ = H et $R_4$ = H,Me, Et, Bu; Y ≠ -C-COOET

(f) quand $R_1 = R_5 = R_6 = R_3 = H$ et $R_4$ = H, Me, Bu; Y ≠ -C-COOMe

comprenant :

a) condensation de l'acide o-nitrobenzoïque ou p-nitrobenzoïque substitué (II)

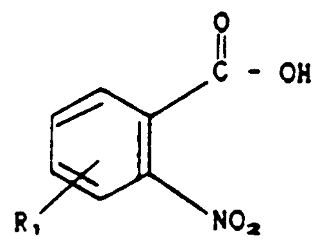

(II)

ou du chlorure correspondant (V)

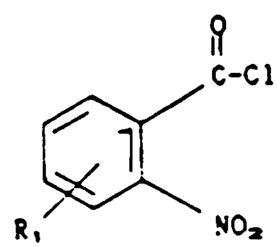

(V)

avec un dérivé 2-aminothiophène, de formule générale (III)

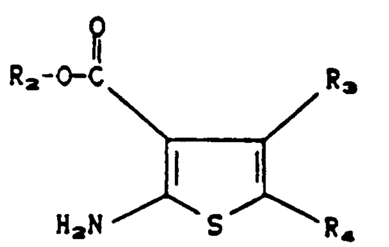

(III)

ou avec un dérivé 2-aminothiazole, de formule générale (IV)

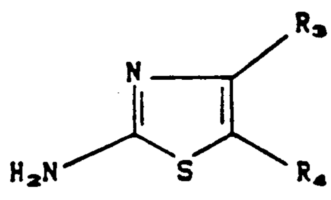

(IV)

b) réduction éventuelle du groupe nitro lié au cycle aromatique ou à un groupe amino et,

c) fonctionnalisation éventuelle de ce groupe amino.

**3.** Procédé tel que revendiqué dans la revendication 2, caractérisé par le fait que la condensation de (V) avec (III) ou avec (IV) est menée dans des solvants organiques aprotiques, en présence de substances basiques, à une températrue comprise entre 40° C et 160° C et, de préférence, entre 40° C et 70° C.

**4.** Procédé tel que revendiqué dans la revendication 3, caractérisé par le fait que les solvants organiques aprotiques sont : $CHCl_3$, $CH_2Cl_2$, acétone, benzène, toluène, THF, dioxane et DMF et sont, de préférence, $CH_2Cl_2$ et $CHCl_3$.

**5.** Procédé tel que revendiqué dans la revendication 3, caractérisé par le fait que les substances basiques sont : $Me_3N$, $Et_3N$, la pyridine, $K_2CO_3$ et $Na_2CO_3$ et sont, de préférence, la triéthylamine et le carbonate de potassium.

**6.** Procédé tel que revendiqué dans la revendication 2, caractérisé par le fait que la réaction de condensation est menée avec un rapport molaire de (V) à (III) ou de (V) à (IV), compris entre 1 et 2,5 et, de préférence, compris entre 1 et 1,2.

**7.** Procédé tel que revendiqué dans la revendication 2, caractérisé par le fait que la condensation de (II) avec (III) ou avec (IV) est menée dans un milieu réactionnel constitué de solvants organiques en présence d'agents de condensation, à une température comprise entre 0° C et 60° C.

**8.** Procédé tel que revendiqué dans la revendication 7, caractérisé par le fait que les solvants organiques utilisés sont : EtOH, THF, le benzène et le dioxane et, de préférence THF.

**9.** Procédé tel que revendiqué dans la revendication 7, caractérisé par le fait que les agents de condensation utilisés sont le dicyclohexylcarbodiimide, l'éthoxyacétylène, le diéthylcyanamide, et le phosphite de triphényle + imidazole, et de préférence le dicyclohexylcarbodiimide.

**10.** Procédé tel que revendiqué dans la revendication 7, caractérisé par le fait que le rapport molaire du composé (II) au composé (III) ou du composé (II) au composé (IV) est compris entre 0,5 et 1 et, de préférence, compris entre 0,5 et 0,67.

**11.** Procédé de préparation de dérivés amides hétérocycliques d'acides benzoïques substitués, de formule générale (I), dans laquelle $R_5$ et $R_6$ forment NH-CO, pour donner des composés tricycliques, caractérisé par le fait que l'on traite avec du phosgène un composé de formule générale (I) dans laquelle $R_5$ et H et $R_6$ est $NH_2$.

**12.** Procédé tel que revendiqué dans la revendication 11, caractérisé par le fait que ce traitement est effectué avec du phosgène dissous dans le toluène.

**13.** Procédé tel que revendiqué dans la revendication 11, caractérisé par le fait que ce traitement est effectué à une température comprise entre la température ambiante et la température d'ébullition à reflux.

**14.** Utilisation de dérivés amides hétérocycliques de l'acide benzoïque substitué, de formule générale (I) :

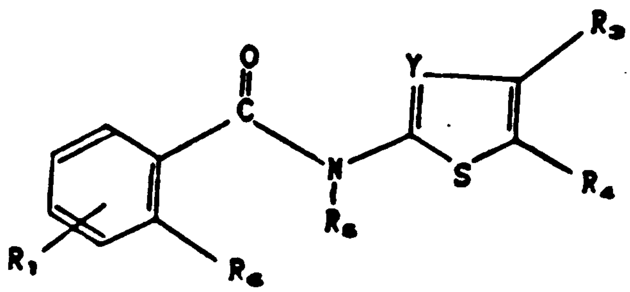

(I)

ou de leurs sels thérapeutiquement actifs,
dans laquelle
$R_1$ est H; 3-$OCH_3$; 3-Cl; 4-Cl; 5-Cl; 3-$CH_3$; 5-$CH_3$; ou 3-OH;
$R_3$ est H; -$CH_3$; -CO-COOH; -CO-$CO_2$-$C_2H_5$;

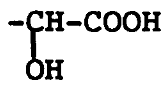 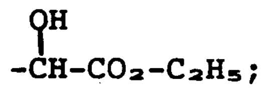

- $CH_2$-COOH; ou -$CH_2$-$CO_2$-$C_2H_5$;
$R_4$ est H; un radical alkyle linéaire ayant de 1 à 4 atomes de carbone; -COOH ou -$CO_2C_2H_5$;
$R_5$ est H, ou $R_5$ et $R_6$ ensemble forment (-CO-NH);
$R_6$ est H; -$NO_2$; -$NH_2$; -NH-COH; -NH-$COCH_3$; -NH-CO-CO-$(CH_2)_n$-$CH_3$ où n vaut 0, 1, 2 ou 3; ou $R_6$ et $R_3$ forment ensemble (NH-CO-);
Y est N; ou C-R dans lequel R est $CO_2R_2$ et $R_2$ est H ou un métal calcalin ou alcalino-terreux équivalent ou un radical alkyle linéaire ou ramifié contenant de 1 à 8 atomes de carbone;
pour la préparation de compositions pharmaceutiques, en vue du traitement des affections allergiques.

**Revendications pour les Etats contractants suivants : AT, ES, GR**

**1.** Procédé de préparation de dérivés amides hétérocycliques d'acides benzoïques substitués, de formule générale (I) :

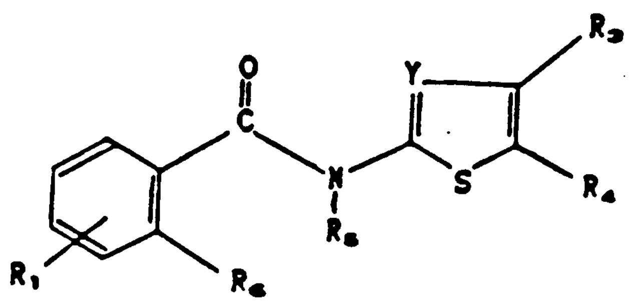

(I)

dans laquelle :
$R_1$ est H; 3-$OCH_3$; 3-Cl; 4-Cl; 5-Cl; 3-$CH_3$; 5-$CH_3$; ou 3-OH;
$R_3$ est H; -$CH_3$; -CO-COOH; -CO-$CO_2$-$C_2H_5$;

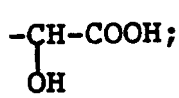 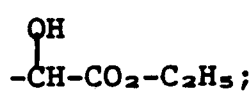

- $CH_2$-COOH; ou -$CH_2$-$CO_2$-$C_2H_5$;
$R_4$ est H; un radical alkyle linéaire ayant de 1 à 4 atomes de carbone; -COOH ou -$CO_2C_2H_5$;
$R_5$ est H, ou $R_5$ et $R_6$ ensemble forment (-CO-NH);
$R_6$ est H; -$NO_2$; -$NH_2$; -NH-COH; -NH-$COCH_3$; -NH-CO-CO-$(CH_2)_n$-$CH_3$ où n vaut 0, 1, 2 ou 3; ou $R_6$ et $R_5$ forment ensemble (NH-CO-);
Y est N; ou C-R dans lequel R est $CO_2R_2$ et $R_2$ est H ou un métal calcalin ou alcalino-terreux équivalent ou un radical alkyle linéaire ou ramifié contenant de 1 à 8 atomes de carbone;
avec les conditions suivantes :
(a) quand $R_1 = R_5 = R_6 = R_3 = R_4 = H$, Y est ≠ N
(b) quand $R_1 = R_3 = R_4 = H$, $R_5$ et $R_6$ forment ensemble -CO-NH-, Y est ≠N
(c) quand $R_1 = R_5 = R_6 = H$, $R_3 = CH_3$ et $R_4 = H$ ou $CH_3$, Y est ≠ -C-COOET
(d) quand $R_1 = R_5 = R_6 = H$ et $R_3 = R_4 = CH_3$, Y est ≠ -C-COOH

(e) quand $R_1 = R_5 = R_6 = H$, $R_3 = H$ et R = H,Me, Et, Bu; Y ≠ -C-COOET

(f) quand $R_1 = R_5 = R_6 = R_3 = H$ et $R_4$ = H, Me, Bu; Y ≠ -C-COOMe

comprenant :

a) condensation de l'acide o-nitrobenzoïque ou p-nitrobenzoïque substitué (II)

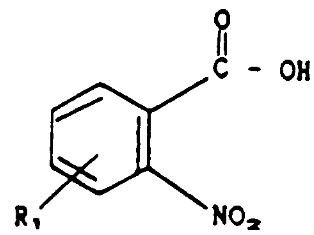

(II)

ou du chlorure correspondant (V)

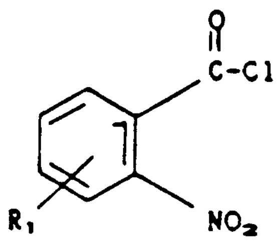

(V)

avec un dérivé 2-aminothiophène, de formule générale (III)

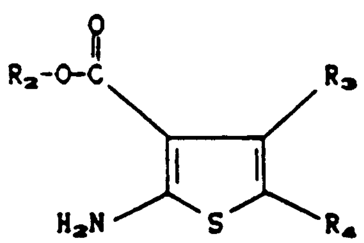

(III)

ou avec un dérivé 2-aminothiazole, de formule générale (IV)

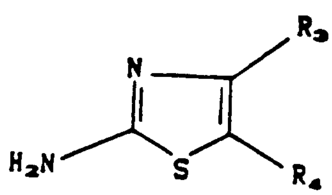

(IV)

b) réduction éventuelle du groupe nitro lié au cycle aromatique ou à un groupe amino et,

c) fonctionnalisation éventuelle de ce groupe amino.

**2.** Procédé tel que revendiqué dans la revendication 1, caractérisé par le fait que la condensation de (V) avec (III) ou avec (IV) est menée dans des solvants organiques aprotiques, en présence de substances basiques, à une températrue comprise entre 40° C et 160° C et, de préférence, entre 40° C et 70° C.

**3.** Procédé tel que revendiqué dans la revendication 2, caractérisé par le fait que les solvants organiques aprotiques sont : $CHCl_3$, $CH_2Cl_2$, acétone, benzène, toluène, THF, dioxane et DMF et sont, de préférence, $CH_2Cl_2$ et $CHCl_3$.

**4.** Procédé tel que revendiqué dans la revendication 2, caractérisé par le fait que les substances basiques sont : $Me_3N$, $Et_3N$, la pyridine, $K_2CO_3$ et $Na_2CO_3$ et sont, de préférence, la triéthylamine et le

carbonate de potassium.

**5.** Procédé tel que revendiqué dans la revendication 1, caractérisé par le fait que la réaction de condensation est menée avec un rapport molaire de (V) à (III) ou de (V) à (IV), compris entre 1 et 2,5 et, de préférence, compris entre 1 et 1,2.

**6.** Procédé tel que revendiqué dans la revendication 1, caractérisé par le fait que la condensation de (II) avec (III) ou avec (IV) est menée dans un milieu réactionnel constitué de solvants organiques en présence d'agents de condensation, à une température comprise entre 0° C et 60° C.

**7.** Procédé tel que revendiqué dans la revendication 6, caractérisé par le fait que les solvants organiques utilisés sont : EtOH, THF, le benzène et le dioxane et, de préférence THF.

**8.** Procédé tel que revendiqué dans la revendication 6, caractérisé par le fait que les agents de condensation utilisés sont le dicyclohexylcarbodiimide, l'éthoxyacétylène, le diéthylcyanamide, et le phosphite de triphényle + imidazole, et de préférence le dicyclohexylcarbodiimide.

**9.** Procédé tel que revendiqué dans la revendication 6, caractérisé par le fait que le rapport molaire du composé (II) au composé (III) ou du composé (II) au composé (IV) est compris entre 0,5 et 1 et, de préférence, compris entre 0,5 et 0,67.

**10.** Procédé de préparation de dérivés amides hétérocycliques d'acides benzoïques substitués, de formule générale (I), dans laquelle $R_5$ et $R_6$ forment NH-CO, pour donner des composés tricycliques, caractérisé par le fait que l'on traite avec du phosgène un composé de formule générale (I) dans laquelle $R_5$ et H et $R_6$ est $NH_2$.

**11.** Procédé tel que revendiqué dans la revendication 10, caractérisé par le fait que ce traitement est effectué avec du phosgène dissous dans le toluène.

**12.** Procédé tel que revendiqué dans la revendication 10, caractérisé par le fait que ce traitement est effectué à une température comprise entre la température ambiante et la température d'ébullition à reflux.

**13.** Utilisation de dérivés amides hétérocycliques de l'acide benzoïque substitué, de formule générale (I) :

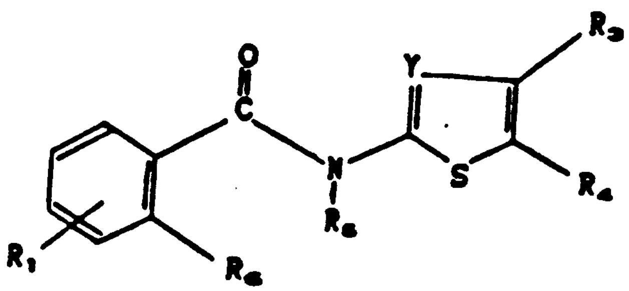

(I)

ou de leurs sels thérapeutiquement actifs,
dans laquelle
$R_1$ est H; 3-$OCH_3$; 3-Cl; 4-Cl; 5-Cl; 3-$CH_3$; 5-$CH_3$; ou 3-OH;
$R_3$ est H; -$CH_3$; -CO-COOH; -CO-$CO_2$-$C_2H_5$;

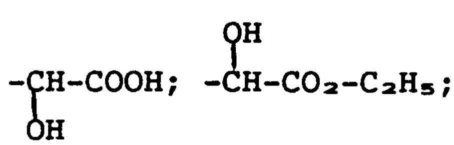

- $CH_2$-COOH; ou -$CH_2$-$CO_2$-$C_2H_5$;

$R_4$ est H; un radical alkyle linéaire ayant de 1 à 4 atomes de carbone; $-COOH$ ou $-CO_2C_2H_5$;
$R_5$ est H, ou $R_5$ et $R_6$ ensemble forment (-CO-NH);
$R_6$ est H; $-NO_2$; $-NH_2$; -NH-COH; $-NH-COCH_3$; $-NH-CO-CO-(CH_2)_n-CH_3$ où n vaut 0, 1, 2 ou 3; ou $R_6$ et $R_3$ forment ensemble (NH-CO-);
Y est N; ou C-R dans lequel R est $CO_2R_2$ et $R_2$ est H ou un métal calcalin ou alcalino-terreux équivalent ou un radical alkyle linéaire ou ramifié contenant de 1 à 8 atomes de carbone;
pour la préparation de compositions pharmaceutiques, en vue du traitement des affections allergiques.

**Patentansprüche**
**Patentansprüche für folgende Vertragsstaaten : BE, CH, DE, FR, GB, IT, LI, LU, NL, SE**

1. Heterocyclische Amidderivate von substituierten Benzoesäuren und therapeutisch annehmbare Salze hievon der allgemeinen Formel

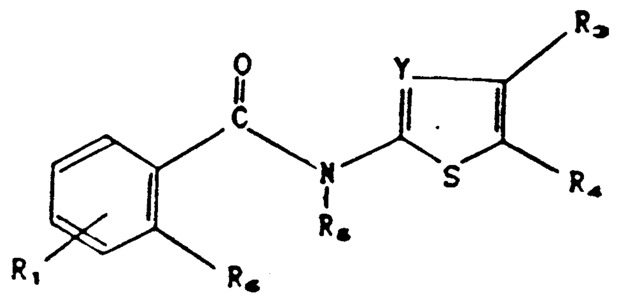

(I),

worin $R_1$ H, $3-OCH_3$, 3-Cl, 4-Cl, 5-Cl, $3-CH_3$, $5-CH_3$ oder 3-OH bedeutet,
$R_3$ H, $-CH_3$, -CO-COOH, $-CO-CO_2-C_2H_5$,

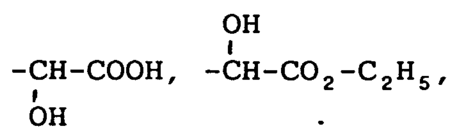

$-CH_2-COOH$ oder $-CH_2-CO_2-C_2H_5$ darstellt,
$R_4$ H, ein linearer Alkylrest mit 1 bis 4 Kohlenstoffatomen, -COOH oder $-CO_2C_2H_5$ ist,
$R_5$ H bedeutet oder $R_5$ und $R_6$ miteinander (-CO-NH) bilden,
$R_6$ die Bedeutung H, $-NO_2$, $-NH_2$, -NH-COH, $-NH-COCH_3$ oder $-NH-CO-CO-(CH_2)_n-CH_3$ hat, wobei n Null, 1, 2 oder 3 ist, oder $R_6$ und $R_5$ miteinander (NH-CO-) bilden,
Y N oder -C-R ist, wobei R die Bedeutung $CO_2R_2$ hat und $R_2$ H oder ein Alkali- oder Erdalkalimetalläquivalent oder ein linearer oder verzweigter Alkylrest mit 1 bis 8 Kohlenstoffatomen ist,
mit den folgenden Maßgaben:
(a) wenn $R_1 = R_5 = R_6 = R_3 = R_4 = H$, $Y \neq N$ ist,
(b) wenn $R_1 = R_3 = R_4 = H$ und $R_5$ und $R_6$ miteinander -CO-NH- bilden, $Y \neq N$ ist,
(c) wenn $R_1 = R_5 = R_6 = H$, $R_3 = CH_3$ und $R_4 = H$ oder $CH_3$, $Y \neq$ -C-COOEt ist,
(d) wenn $R_1 = R_5 = R_6 = H$ und $R_3 = R_4 = CH_3$, $Y \neq$ -C-COOH ist,
(e) wenn $R_1 = R_5 = R_6 = H$, $R_3 = H$ und $R_4 = H$, Me, Et oder Bu, $Y \neq$ -C-COOEt ist,
(f) wenn $R_1 = R_5 = R_6 = R_3 = H$ und $R_4 = H$, Me oder Bu, $Y \neq$ -C-COOMe ist.

2. Verfahren zum Herstellen von heterocyclischen Amidderivaten von substituierten Benzoesäuren der allgemeinen Formel (I)

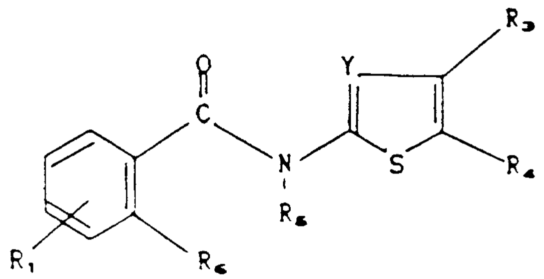

(I),

worin $R_1$ H, 3-$OCH_3$, 3-Cl, 4-Cl, 5-Cl, 3-$CH_3$, 5-$CH_3$ oder 3-OH bedeutet,

$R_3$ H, -$CH_3$, -CO-COOH, -CO-$CO_2$-$C_2H_5$,

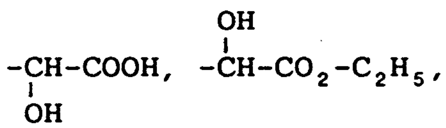

-$CH_2$-COOH oder -$CH_2$-$CO_2$-$C_2H_5$ darstellt,

$R_4$ H, ein linearer Alkylrest mit 1 bis 4 Kohlenstoffatomen, -COOH oder -$CO_2C_2H_5$ ist,

$R_5$ H bedeutet oder $R_5$ und $R_6$ miteinander (-CO-NH) bilden,

$R_6$ die Bedeutung H, -$NO_2$, -$NH_2$, -NH-COH, -NH-$COCH_3$ oder -NH-CO-CO-$(CH_2)_n$-$CH_3$ hat, wobei n Null, 1, 2 oder 3 ist, oder $R_6$ und $R_5$ miteinander (NH-CO-) bilden,

Y N oder -C-R ist, wobei R die Bedeutung $CO_2R_2$ hat und $R_2$ H oder ein Alkali- oder Erdalkalimetalläquivalent oder ein linearer oder verzweigter Alkylrest mit 1 bis 8 Kohlenstoffatomen ist,

mit den folgenden Maßgaben:

(a) wenn $R_1 = R_5 = R_6 = R_3 = R_4 = H$, $Y \neq N$ ist,

(b) wenn $R_1 = R_3 = R_4 = H$ und $R_5$ und $R_6$ miteinander -CO-NH- bilden, $Y \neq N$ ist,

(c) wenn $R_1 = R_5 = R_6 = H$, $R_3 = CH_3$ und $R_4 = H$ oder $CH_3$, $Y \neq$ -C-COOEt ist,

(d) wenn $R_1 = R_5 = R_6 = H$ und $R_3 = R_4 = CH_3$, $Y \neq$ -C-COOH ist,

(e) wenn $R_1 = R_5 = R_6 = H$, $R_3 = H$ und $R_4 = H$, Me, Et oder Bu, $Y \neq$ -C-COOEt ist,

(f) wenn $R_1 = R_5 = R_6 = R_3 = H$ und $R_4 = H$, Me oder Bu, $Y \neq$ -C-COOMe ist,

umfassend

(a) das Kondensieren von substituierter o-Nitrobenzoe- oder p-Nitrobenzoesäure (II)

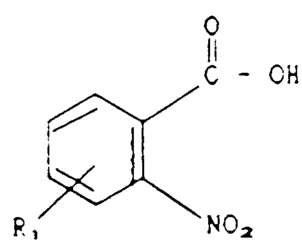

(II)

oder des entsprechenden Chlorids (V)

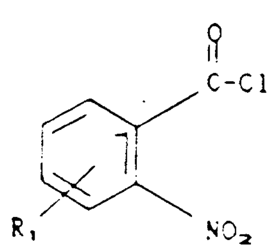

(V)

mit einem 2-Aminothiophenderivat der allgemeinen Formel (III)

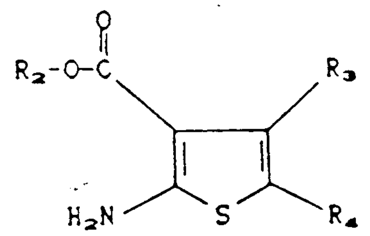

(III)

oder einem 2-Aminothiazolderivat der allgemeinen Formel (IV)

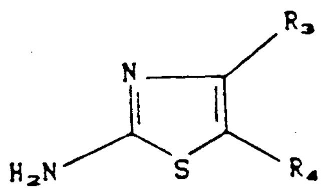

(IV),

b) gegebenenfalls das Reduzieren der an den aromatischen Ring gebundenen Nitrogruppe zu einer Aminogruppe und

c) gegebenenfalls das Funktionalisieren dieser Aminogruppe.

**3.** Verfahren nach Anspruch 2, dadurch gekennzeichnet, daß die Kondensation von (V) mit (III) oder mit (IV) in aprotischen organischen Lösungsmitteln in Anwesenheit von basischen Substanzen bei einer Temperatur von 40 bis 160 ° C, vorzugsweise 40 bis 70 ° C, durchgeführt wird.

**4.** Verfahren nach Anspruch 3, dadurch gekennzeichnet, daß die aprotischen organischen Lösungsmittel $CHCl_3$, $CH_2Cl_2$, Aceton, Benzol, Toluol, THF, Dioxan und DMF, vorzugsweise $CH_2Cl_2$ und $CHCl_3$, sind.

**5.** Verfahren nach Anspruch 3, dadurch gekennzeichnet, daß die basischen Substanzen $Me_3N$, $Et_3N$, Pyridin, $K_2CO_3$ und $Na_2CO_3$, vorzugsweise Triäthylamin und Kaliumcarbonat, sind.

**6.** Verfahren nach Anspruch 2, dadurch gekennzeichnet, daß die Kondensationsreaktion mit einem Molverhältnis von (V) zu (III) oder von (V) zu (IV) von 1 bis 2,5, vorzugsweise 1 bis 1,2, durchgeführt wird.

**7.** Verfahren nach Anspruch 2, dadurch gekennzeichnet, daß die Kondensation von (II) mit (III) oder mit (IV) in einem Reaktionsmedium bestehend aus organischen Lösungsmitteln in Anwesenheit von Kondensationsmitteln bei einer Temperatur von 0 bis 60 ° C durchgeführt wird.

**8.** Verfahren nach Anspruch 7, dadurch gekennzeichnet, daß die verwendeten organischen Lösungsmittel EtOH, THF, Benzol und Dioxan, vorzugsweise THF, sind.

**9.** Verfahren nach Anspruch 7, dadurch gekennzeichnet, daß die verwendeten Kondensationsmittel Dicyclohexylcarbodiimid, Äthoxyacetylen, Diäthylcyanamid und Triphenylphosphit + Imidazol, vorzugsweise Dicyclohexylcarbodiimid, sind.

**10.** Verfahren nach Anspruch 7, dadurch gekennzeichnet, daß das Molverhältnis der Verbindung (II) zur Verbindung (III) oder der Verbindung (II) zur Verbindung (IV) 0,5 bis 1, vorzugsweise 0,5 bis 0,67, beträgt.

**11.** Verfahren zum Herstellen von heterocyclischen Amidderivaten von substituierten Benzoesäuren der allgemeinen Formel (I), worin $R_5$ und $R_6$ NH-CO bilden, wobei tricyclische Verbindungen erhalten werden, dadurch gekennzeichnet, daß eine Verbindung der allgemeinen Formel (I), worin $R_5$ H ist und $R_6$ $NH_2$ bedeutet, mit Phosgen behandelt wird.

**12.** Verfahren nach Anspruch 11, dadurch gekennzeichnet, daß die Behandlung mit in Toluol gelöstem Phosgen durchgeführt wird.

**13.** Verfahren nach Anspruch 11, dadurch gekennzeichnet, daß die Behandlung bei einer Temperatur von Umgebungstemperatur bis zur Rückflußsiedetemperatur durchgeführt wird.

**14.** Verwendung von heterocyclischen Amidderivaten von substituierter Benzoesäure der allgemeinen Formel (I)

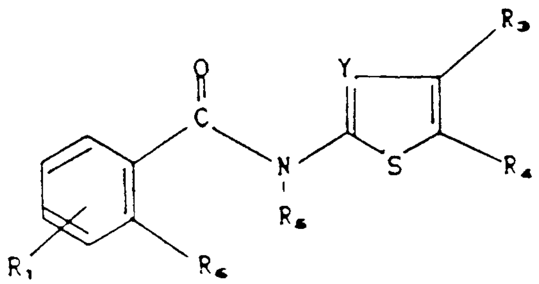

(I)

und ihrer therapeutisch aktiven Salze,
worin $R_1$ H, 3-$OCH_3$, 3-Cl, 4-Cl, 5-Cl, 3-$CH_3$, 5-$CH_3$ oder 3-OH bedeutet,
$R_3$ H, -$CH_3$, -CO-COOH, -CO-$CO_2$-$C_2H_5$,

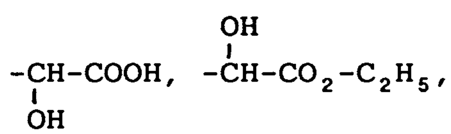

-$CH_2$-COOH oder -$CH_2$-$CO_2$-$C_2H_5$ darstellt,
$R_4$ H, ein linearer Alkylrest mit 1 bis 4 Kohlenstoffatomen, -COOH oder -$CO_2C_2H_5$ ist,
$R_5$ H bedeutet oder $R_5$ und $R_6$ miteinander (-CO-NH) bilden,
$R_6$ die Bedeutung H, -$NO_2$, -$NH_2$, -NH-COH, -NH-$COCH_3$ oder -NH-CO-CO-$(CH_2)_n$-$CH_3$ hat, wobei n Null, 1, 2 oder 3 ist, oder $R_6$ und $R_5$ miteinander (NH-CO-) bilden,
Y N oder -C-R ist, wobei R die Bedeutung $CO_2R_2$ hat und $R_2$ H oder ein Alkali- oder Erdalkalimetalläquivalent oder ein linearer oder verzweigter Alkylrest mit 1 bis 8 Kohlenstoffatomen ist,
zum Herstellen einer pharmazeutischen Zusammensetzung zur Behandlung von allergischen Erkrankungen.

**Patentansprüche für folgende Vertragsstaaten : AT, ES, GR**

**1.** Verfahren zum Herstellen von heterocyclischen Amidderivaten von substituierten Benzoesäuren der allgemeinen Formel (I)

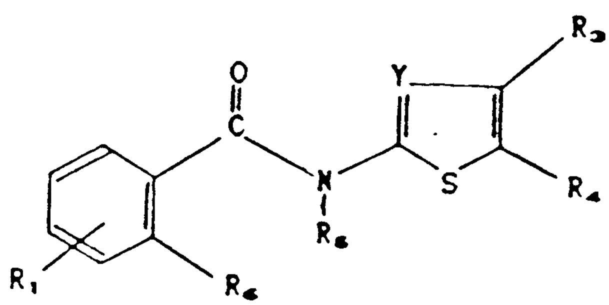

(I),

worin $R_1$ H, 3-$OCH_3$, 3-Cl, 4-Cl, 5-Cl, 3-$CH_3$, 5-$CH_3$ oder 3-OH bedeutet,
$R_3$ H, -$CH_3$, -CO-COOH, -CO-$CO_2$-$C_2H_5$,

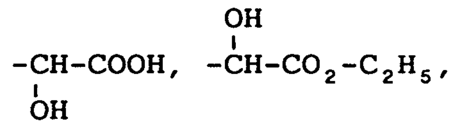

$-CH_2-COOH$ oder $-CH_2-CO_2-C_2H_5$ darstellt,

$R_4$ H, ein linearer Alkylrest mit 1 bis 4 Kohlenstoffatomen, -COOH oder $-CO_2C_2H_5$ ist,

$R_5$ H bedeutet oder $R_5$ und $R_6$ miteinander (-CO-NH) bilden,

$R_6$ die Bedeutung H, $-NO_2$, $-NH_2$, -NH-COH, $-NH-COCH_3$ oder $-NH-CO-CO-(CH_2)_n-CH_3$ hat, wobei n Null, 1, 2 oder 3 ist, oder $R_6$ und $R_5$ miteinander (NH-CO-) bilden,

Y N oder -C-R ist, wobei R die Bedeutung $CO_2R_2$ hat und $R_2$ H oder ein Alkali- oder Erdalkalimetalläquivalent oder ein linearer oder verzweigter Alkylrest mit 1 bis 8 Kohlenstoffatomen ist,

mit den folgenden Maßgaben:

(a) wenn $R_1 = R_5 = R_6 = R_3 = R_4 = H$, $Y \neq N$ ist,

(b) wenn $R_1 = R_3 = R_4 = H$ und $R_5$ und $R_6$ miteinander -CO-NH- bilden, $Y \neq N$ ist,

(c) wenn $R_1 = R_5 = R_6 = H$, $R_3 = CH_3$ und $R_4 = H$ oder $CH_3$, $Y \neq$ -C-COOEt ist,

(d) wenn $R_1 = R_5 = R_6 = H$ und $R_3 = R_4 = CH_3$, $Y \neq$ -C-COOH ist,

(e) wenn $R_1 = R_5 = R_6 = H$, $R_3 = H$ und $R_4 = H$, Me, Et oder Bu, $Y \neq$ -C-COOEt ist,

(f) wenn $R_1 = R_5 = R_6 = R_3 = H$ und $R_4 = H$, Me oder Bu, $Y \neq$ -C-COOMe ist,

umfassend

(a) das Kondensieren von substituierter o-Nitrobenzoe- oder p-Nitrobenzoesäure (II)

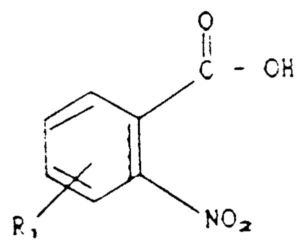

(II)

oder des entsprechenden Chlorids (V)

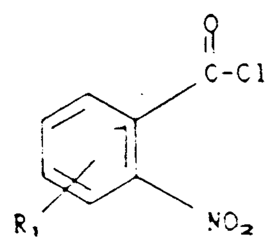

(V)

mit einem 2-Aminothiophenderivat der allgemeinen Formel (III)

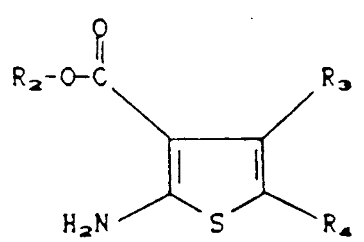

(III)

oder einem 2-Aminothiazolderivat der allgemeinen Formel (IV)

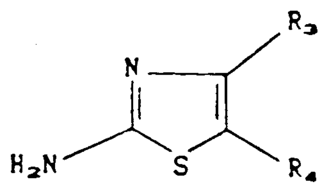

(IV),

b) gegebenenfalls das Reduzieren der an den aromatischen Ring gebundenen Nitrogruppe zu einer Aminogruppe und
c) gegebenenfalls das Funktionalisieren dieser Aminogruppe.

**2.** Verfahren nach Anspruch 1, dadurch gekennzeichnet, daß die Kondensation von (V) mit (III) oder mit (IV) in aprotischen organischen Lösungsmitteln in Anwesenheit von basischen Substanzen bei einer Temperatur von 40 bis 160 ° C, vorzugsweise 40 bis 70 ° C, durchgeführt wird.

**3.** Verfahren nach Anspruch 2, dadurch gekennzeichnet, daß die aprotischen organischen Lösungsmittel $CHCl_3$, $CH_2Cl_2$, Aceton, Benzol, Toluol, THF, Dioxan und DMF, vorzugsweise $CH_2Cl_2$ und $CHCl_3$, sind.

**4.** Verfahren nach Anspruch 2, dadurch gekennzeichnet, daß die basischen Substanzen $Me_3N$, $Et_3N$, Pyridin, $K_2CO_3$ und $Na_2CO_3$, vorzugsweise Triäthylamin und Kaliumcarbonat, sind.

**5.** Verfahren nach Anspruch 1, dadurch gekennzeichnet, daß die Kondensationsreaktion mit einem Molverhältnis von (V) zu (III) oder von (V) zu (IV) von 1 bis 2,5, vorzugsweise 1 bis 1,2, durchgeführt wird.

**6.** Verfahren nach Anspruch 1, dadurch gekennzeichnet, daß die Kondensation von (II) mit (III) oder mit (IV) in einem Reaktionsmedium bestehend aus organischen Lösungsmitteln in Anwesenheit von Kondensationsmitteln bei einer Temperatur von 0 bis 60 ° C durchgeführt wird.

**7.** Verfahren nach Anspruch 6, dadurch gekennzeichnet, daß die verwendeten organischen Lösungsmittel EtOH, THF, Benzol und Dioxan, vorzugsweise THF, sind.

**8.** Verfahren nach Anspruch 6, dadurch gekennzeichnet, daß die verwendeten Kondensationsmittel Dicyclohexylcarbodiimid, Äthoxyacetylen, Diäthylcyanamid und Triphenylphosphit + Imidazol, vorzugsweise Dicyclohexylcarbodiimid, sind.

**9.** Verfahren nach Anspruch 6, dadurch gekennzeichnet, daß das Molverhältnis der Verbindung (II) zur Verbindung (III) oder der Verbindung (II) zur Verbindung (IV) 0,5 bis 1, vorzugsweise 0,5 bis 0,67, beträgt.

**10.** Verfahren zum Herstellen von heterocyclischen Amidderivaten von substituierten Benzoesäuren der allgemeinen Formel (I), worin $R_5$ und $R_6$ NH-CO bilden, wobei tricyclische Verbindungen erhalten werden, dadurch gekennzeichnet, daß eine Verbindung der allgemeinen Formel (I), worin $R_5$ H ist und $R_6$ $NH_2$ bedeutet, mit Phosgen behandelt wird.

**11.** Verfahren nach Anspruch 10, dadurch gekennzeichnet, daß die Behandlung mit in Toluol gelöstem Phosgen durchgeführt wird.

**12.** Verfahren nach Anspruch 10, dadurch gekennzeichnet, daß die Behandlung bei einer Temperatur von Umgebungstemperatur bis zur Rückflußsiedetemperatur durchgeführt wird.

**13.** Verwendung von heterocyclischen Amidderivaten von substituierter Benzoesäure der allgemeinen Formel (I)

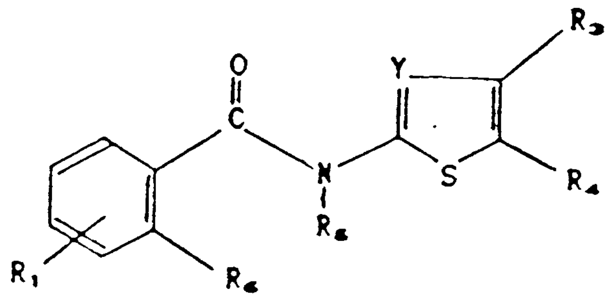

(I)

und ihrer therapeutisch aktiven Salze,
worin $R_1$ H, 3-$OCH_3$, 3-Cl, 4-Cl, 5-Cl, 3-$CH_3$, 5-$CH_3$ oder 3-OH bedeutet,
$R_3$ H, -$CH_3$, -CO-COOH, -CO-$CO_2$-$C_2H_5$,

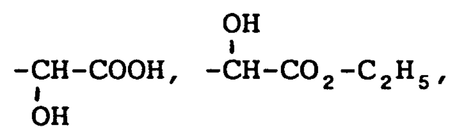

-$CH_2$-COOH oder -$CH_2$-$CO_2$-$C_2H_5$ darstellt,
$R_4$ H, ein linearer Alkylrest mit 1 bis 4 Kohlenstoffatomen, -COOH oder -$CO_2C_2H_5$ ist,
$R_5$ H bedeutet oder $R_5$ und $R_6$ miteinander (-CO-NH) bilden,
$R_6$ die Bedeutung H, -$NO_2$, -$NH_2$, -NH-COH, -NH-$COCH_3$ oder -NH-CO-CO-$(CH_2)_n$-$CH_3$ hat, wobei n Null, 1, 2 oder 3 ist, oder $R_6$ und $R_5$ miteinander (NH-CO-) bilden,
Y N oder -C-R ist, wobei R die Bedeutung $CO_2R_2$ hat und $R_2$ H oder ein Alkali- oder Erdalkalimetalläquivalent oder ein linearer oder verzweigter Alkylrest mit 1 bis 8 Kohlenstoffatomen ist,
zum Herstellen einer pharmazeutischen Zusammensetzung zur Behandlung von allergischen Erkrankungen.